(19) Europäisches Patentamt / European Patent Office / Office européen des brevets

(11) **EP 3 058 345 B1**

(12) **FASCICULE DE BREVET EUROPEEN**

(45) Date de publication et mention de la délivrance du brevet:
**17.02.2021 Bulletin 2021/07**

(21) Numéro de dépôt: **14793464.0**

(22) Date de dépôt: **17.10.2014**

(51) Int Cl.:
**G01N 21/45** *(2006.01)*          **G01N 33/50** *(2006.01)*
**G02B 1/11** *(2015.01)*          **G02B 21/34** *(2006.01)*

(86) Numéro de dépôt international:
**PCT/EP2014/072307**

(87) Numéro de publication internationale:
**WO 2015/055809 (23.04.2015 Gazette 2015/16)**

(54) **SUPPORTS AMPLIFICATEURS DE CONTRASTE POUR L'OBSERVATION D'UN ECHANTILLON, LEUR PROCEDES DE FABRICATION ET LEURS UTILISATIONS**

KONTRASTVERSTÄRKUNGSTRÄGER ZUR BEOBACHTUNG EINER PROBE, HERSTELLUNGSVERFAHREN DAFÜR UND VERWENDUNGEN DAVON

CONTRAST AMPLIFYING SUPPORT FOR THE OBSERVATION OF A SAMPLE, PRODUCTION METHODS THEREOF AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.10.2013 FR 1360192**

(43) Date de publication de la demande:
**24.08.2016 Bulletin 2016/34**

(73) Titulaires:
• **Centre National de la Recherche Scientifique**
**75016 Paris (FR)**
• **Universite du Maine**
**72085 Le Mans Cedex 9 (FR)**

(72) Inventeurs:
• **AUSSERRE, Dominique**
**F-72270 Soulitre (FR)**
• **AMRA, Claude**
**F-13013 Marseille (FR)**
• **ZERRAD, Myriam**
**F-13001 Marseille (FR)**
• **ABOU KHACHFE, Refahi**
**BP 813**
**Saida (LB)**

(74) Mandataire: **Marks & Clerk France**
**Immeuble "Visium"**
**22, avenue Aristide Briand**
**94117 Arcueil Cedex (FR)**

(56) Documents cités:
**WO-A1-92/02364          WO-A1-2006/013287**
**WO-A1-2014/016813     US-A- 5 812 405**

**Description**

L'invention porte sur des supports amplificateurs de contraste pour l'observation d'un échantillon, ainsi que sur des procédés de fabrication de tels supports. L'invention porte également sur des procédés d'observation d'échantillons et des procédés de détection ou dosage d'espèces chimiques ou biologiques mettant en œuvre de tels supports.

L'invention est susceptible d'être appliquée à différents domaines techniques, tels que la biologie (détection de biomolécules ou microorganismes, observation de cultures cellulaires), les nanotechnologies (visualisation de nano-objets, tels que des nanotubes), la microélectronique, la science des matériaux, etc.

L'utilisation de couches antireflet (ou couches « λ/4 ») pour augmenter le contraste optique d'un objet observé en microscopie optique par réflexion est une technique connue depuis de nombreuses années et très puissante ; elle a permis notamment la première observation de marches moléculaires par Langmuir et Blodgett en 1937 et, plus récemment, la visualisation des couches de graphène par Novoselov et al.

Soit $I$ l'intensité lumineuse réfléchie par l'objet à observer, déposé sur un support, et $I_s$ celle réfléchie par le support seul ; alors, le contraste avec lequel l'échantillon est observé vaut $C=(I-I_s)/(I+I_s)$. On comprend que la valeur absolue de ce contraste prend sa valeur maximale (égale à 1) quand $I_s=0$, c'est-à-dire quand le support a une réflectivité nulle, ou bien quand l'objet supporté à une réflectivité nulle. Dans le cas le plus simple, la condition $I_s=0$ est satisfaite en utilisant en tant que support un substrat transparent sur lequel est déposée une couche mince, également transparente, dont l'épaisseur et l'indice de réfraction sont choisis de manière opportune. Dans le cas d'une couche antireflet unique, éclairée en incidence normale avec un milieu incident (dont provient l'éclairage) et un milieu émergent (le substrat) transparents et semi-infinis, on obtient les conditions suivantes :

$$n_1{}^2 = n_0 n_3 \qquad\qquad\qquad (1a)$$

$$n_1 e_1 = \lambda/4 \qquad\qquad\qquad (1b)$$

où $n_1$ est l'indice de réfraction (réel) de la couche, $n_0$ et $n_3$ les indices de réfraction (également réels) des milieux incident et émergent, $e_1$ l'épaisseur de la couche et $\lambda$ la longueur d'onde d'éclairage.

Pour des milieux incident et émergent donnés, l'équation (1a) détermine de manière univoque l'indice de réfraction de la couche antireflet. Malheureusement, cet indice peut ne pas correspondre à un matériau d'usage courant, ou satisfaisant à diverses contraintes liées à l'application spécifiquement considérée. Par exemple, dans le cas d'une interface air-verre - dont l'intérêt pratique est évident - on obtient $n_1 \cong 1{,}27$, ce qui nécessite l'utilisation de matériaux composites tels que des aérogels.

L'invention vise à surmonter cet inconvénient de l'art antérieur.

Pour y parvenir, l'invention propose d'utiliser des couches antireflets absorbantes, présentant un indice de réfraction complexe. Le degré de liberté additionnel associé à la présence d'une partie imaginaire de l'indice permet de relâcher la contrainte pesant sur la valeur de sa partie réelle. Par ailleurs, alors qu'il est difficile de modifier la partie réelle de l'indice de réfraction d'un matériau, il est relativement simple de modifier sa partie imaginaire (par exemple, en introduisant des impuretés absorbantes ou diffusantes, la diffusion « simulant » une absorption).

Il convient de noter que - dans le cas des couches « λ/4 » conventionnelles - l'augmentation du contraste résulte d'un effet interférentiel qui met en jeu des réflexions multiples aux interfaces milieu incident/couche et couche/milieu émergent. Or, l'absorption de la lumière à l'intérieur de la couche tend à supprimer l'interférence entre ces réflexions multiples. Par conséquent, le concept même d'une « couche antireflet absorbante » parait de prime abord contraire à l'intuition.

L'article de S. G. Moiseev et S. V. Vinogradov « Design of Antireflection Composite Coating Based on Metal Nanoparticle », Physics of Wave Phenomena, 2011, Vol 10, N°1, pages 47 - 51 étudie les conditions que doit satisfaire une couche mince faiblement absorbante déposée sur un substrat transparent pour annuler la réflexion en incidence normale à l'interface air-substrat, l'éclairage étant effectué par l'air. Ce document décrit également une couche mince absorbante en matériau composite contenant des nanoparticules métalliques satisfaisant de manière approchée à ces conditions. Cette couche réduit la réflexion à l'interface air-substrat, mais ne l'annule pas totalement. En outre, son fonctionnement a été démontré - par une étude analytique limitée aux matériaux à très faible absorption, mais ce résultat est difficilement généralisable. Par ailleurs, un tel revêtement n'est pas destiné à réaliser un support amplificateur de contraste.

Les articles suivants :

- M. A. Kats et al. « Nanometre optical coatings based on strong interference effects in higly absorbing média », Nature Materials, Vol. 12, Janvier 2013, pages 20 - 24; et
- R. M. A. Azzam et al. « Antieflection of an absorbing substrate by an absorbing thin film at normal incidence », Applied Optics, Vol 26, No 4, pages 719 - 722 (1987)

divulguent des couches antireflet absorbantes déposées sur des substrats qui sont à leur tour absorbants. Là encore, seuls des cas particuliers sont décrits, qui sont difficilement généralisables. En outre, dans le cas de l'article de M. A. Kats et al., la suppression de la réflexion n'est que partielle.

Le document US 5,216,542 divulgue un revêtement antireflet pour un substrat en verre comportant, sur une face avant du substrat (destinée à être éclairé), une structure multicouches comprenant des couches transparentes et des couches absorbantes en $TiN_x$ et, sur une face arrière dudit substrat, une couche absorbante unique en $TiN_x$ dont l'épaisseur n'est cependant pas de nature à assurer une réflectivité nulle, mais seulement faible. Un tel revêtement n'est pas destiné à réaliser un support amplificateur de contraste.

Conformément à l'invention, le support amplificateur de contraste comprenant une couche antireflet absorbante est dimensionné de manière à être utilisé dans une configuration « inversée » ou « face arrière », c'est-à-dire avec éclairage et observation à travers le substrat qui a un indice de réfraction supérieur à celui du milieu émergent (ou « milieu ambiant »). Cette configuration est particulièrement adaptée lorsque le substrat forme une fenêtre d'observation et la couche antireflet absorbante est mise en contact avec un milieu aqueux (applications chimiques ou biologiques) ou maintenue dans une enceinte sous vide ou à atmosphère contrôlée (applications telles que les procédés de dépôt). Elle est opposée à celle décrite dans l'article précité de S. G. Moiseev et S. V. Vinogradov. En outre, l'invention permet de réaliser des supports adaptés à pratiquement tout milieu ambiant transparent, et pas uniquement à l'air.

Par ailleurs, des couches antireflet absorbantes telles que décrites ci-après peuvent également convenir à des applications autres que l'amplification de contraste - en fait, à chaque fois qu'on souhaite supprimer ou atténuer la réflexion de la lumière entre un substrat transparent et un milieu ambiant à plus faible indice, en présence d'un éclairage provenant dudit substrat.

Un objet de l'invention est donc un support amplificateur de contraste | tel que défini dans la revendication 1. De préférence ladite couche n'est pas en nitrure de titane ($TiN_x$).

Un autre objet de l'invention est un procédé de fabrication d'un support amplificateur de tel que défini dans la revendication 8.

Encore un autre objet de l'invention est un procédé d'observation d'un échantillon comportant les étapes suivantes :

A. Disposer ledit échantillon sur une couche en matériau absorbant d'épaisseur $e_1$ présentant un indice de réfraction complexe $N_1=n_1-jk_1$, comprise entre un premier milieu transparent, dit incident, ayant un indice de réfraction réel $n_0$ et un deuxième milieu transparent, dit émergent, ayant un indice de réfraction réel $n_3<n_0$ ;

B. éclairer ledit échantillon en incidence normale au moins à ladite longueur d'onde d'éclairage $\lambda$ à travers ledit milieu incident ;

C. observer l'échantillon ainsi éclairé, également à travers-ledit milieu incident ;

dans lequel ladite couche en matériau absorbant présente un indice de réfraction complexe et une épaisseur tels que :

a)

$$\nu_1{}^2 = 1 + \kappa_1{}^2 \; ;$$

b)

$$\delta_1 = \frac{(n_0/n_3-1)}{2\nu_1\kappa_1}\left[1 - e^{-\frac{\kappa_1}{K}}\right] \qquad ;$$

et

c) $k_1 \geq 0{,}01$, et de manière encore préférée $k_1 \geq 0{,}1$

où :

$$\delta_1 = \frac{2\pi n_0}{\lambda} e_1 ;$$

$$\nu_1 = \frac{n_1}{\sqrt{n_0 n_3}} ;$$

$$\kappa_1 = \frac{k_1}{\sqrt{n_0 n_3}} ;$$

et

$$K = \left\{ [\pi/(n_0/n_3 - 1)]\sqrt{n_0/n_3} \right\}^{-1}$$

avec une tolérance inférieure ou égale à 5%, et de préférence inférieure ou égale à 0,3%, pour $n_0$, $n_1$ et $k_1$ et avec une tolérance inférieure ou égale à 30% et de préférence inférieure ou égale à 5% pour $e_1$.

Encore un autre objet de l'invention est un procédé de détection ou dosage d'au moins une espèce chimique ou biologique comportant les étapes suivantes :

I. procurer un support amplificateur de contraste tel que mentionné plus haut, comprenant une couche ou surface fonctionnalisée, susceptible de fixer au moins une espèce chimique ou biologique ;
II. placer ladite couche ou surface fonctionnalisée en contact avec au moins une solution contenant une espèce chimique ou biologique marquée par des nanoparticules métalliques ou un marqueur absorbant ou diffusant, ladite espèce étant susceptible de se fixer sur ladite couche ou surface fonctionnalisée, soit directement, soit par l'intermédiaire d'une ou plusieurs autres espèces chimiques ou biologiques, moyennant quoi lesdites particules forment une couche métallique, absorbante ou diffusante continue ou discontinue ;
III. éclairer ledit support amplificateur de contraste en incidence normale au moins à ladite longueur d'onde d'éclairage $\lambda$ à travers ledit substrat ;
IV. observer ledit support amplificateur de contraste ainsi éclairé, également à travers ledit substrat.

Encore un autre objet de l'invention est un procédé de détection ou dosage d'au moins une espèce chimique ou biologique comportant les étapes suivantes :

I. procurer un substrat transparent portant une couche ou surface fonctionnalisée, susceptible de fixer au moins une espèce chimique ou biologique ;
II. placer ladite couche ou surface fonctionnalisée en contact avec au moins une solution contenant une espèce chimique ou biologique marquée par des nanoparticules métalliques ou un marqueur absorbant ou diffusant, ladite espèce étant susceptible de se fixer sur ladite couche ou surface fonctionnalisée - soit directement, soit par l'intermédiaire d'une ou plusieurs autres espèces chimiques ou biologiques - pour former une couche métallique, absorbante ou diffusante continue ou discontinue, ledit substrat transparent formant, avec ladite couche ou surface fonctionnalisée et la couche métallique, absorbante ou diffusante ainsi formée, un support amplificateur de contraste tel que mentionné plus haut ;
III. éclairer ledit support amplificateur de contraste en incidence normale au moins à ladite longueur d'onde d'éclairage $\lambda$ à travers ledit substrat ;
IV. observer ledit support amplificateur de contraste ainsi éclairé, également à travers ledit substrat.

Des modes de réalisation particuliers d'un tel support et de tels procédés constituent l'objet des revendications dépendantes.
Conventionnellement, on considérera qu'un matériau est transparent à une longueur d'onde $\lambda$ lorsque la partie imaginaire de son indice de réfraction à cette longueur d'onde est inférieure à 0,01, voire à 0,001, voire à 0,0001.
D'autres caractéristiques, détails et avantages de l'invention ressortiront à la lecture de la description faite en référence aux dessins annexés donnés à titre d'exemple et qui représentent, respectivement :

- La figure 1, une structure constituée par une couche mince entre deux milieu semi-infinis ;
- Les figures 2A à 2D, des graphiques illustrant la relation entre les parties réelles et imaginaires des indices de réfraction de couches antireflet absorbantes selon différents modes de réalisation de l'invention ;
- Les figures 3A à 3C, des graphiques illustrant la relation entre les épaisseurs et les parties imaginaires des indices de réfraction de couches antireflet absorbantes selon différents modes de réalisation de l'invention ;
- Les figures 4A à 4F, des graphiques illustrant la relation entre les épaisseurs et les réflectances (4A - 4D) ou absorbances (4E, 4F) de couches antireflet absorbantes selon différents modes de réalisation de l'invention ;
- Les figures 5A et 5B, des graphiques illustrant les contrastes d'observation d'un échantillon obtenus grâce à des supports selon différents modes de réalisation de l'invention ;
- Les figures 5C et 5D, des graphiques illustrant les contrastes d'observation d'échantillons d'épaisseurs différentes obtenus grâce à un support selon un mode de réalisation de l'invention ;
- La figure 6, une application d'un support amplificateur de contraste selon un mode de réalisation de l'invention ;
- Les figures 7A à 7E, des procédés de détection ou dosage d'au moins une espèce chimique ou biologique selon différents modes de réalisation de l'invention ; et
- La figure 8, un support amplificateur de contraste illustré à titre d'exemple mais ne relevant pas de l'invention, mettant en œuvre un substrat absorbant.

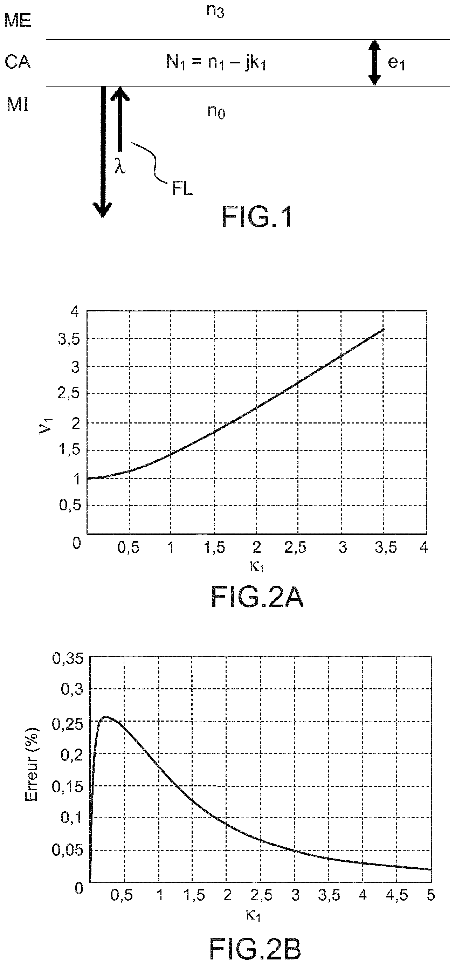

La figure 1 illustre un faisceau de lumière parallèle FL (pouvant être assimilé localement à une onde plane) et monochromatique à une longueur d'onde (dans le vide) $\lambda$, en incidence normale sur une structure constituée par : un milieu semi-infini dit incident MI, dont provient le faisceau de lumière, transparent et caractérisé par un indice de réfraction réel $n_0$ ; une couche en matériau absorbant CA d'épaisseur $e_1$, caractérisée par un indice de réfraction complexe $N_1 = n_1 - jk_1$ (« j » étant l'unité imaginaire) ; et un milieu semi-infini dit émergent ME, situé du côté de la couche opposé à celui dont provient la lumière, transparent et caractérisé par un indice de réfraction réel $n_3 < n_0$. Le milieu incident peut notamment être un substrat, par exemple en verre, sur lequel est déposée la couche CA. Un échantillon (non représenté) d'indice de réfraction réel $n_2$ - ou d'indice de réfraction complexe $N_2 = n_2 - jk_2$ - peut être déposé sur la couche CA, du côté du milieu émergent. Comme cela a été expliqué plus haut, afin de maximiser le contraste avec lequel l'échantillon est observé, il faut annuler la réflectance de l'ensemble milieu incident MI / couche CA / milieu émergent ME en l'absence d'échantillon.

Le coefficient de réflexion complexe d'une structure du type illustré sur la figure 1 (couche d'épaisseur $e_1$ comprise entre deux milieux semi-infinis) est donné par la formule d'Airy :

$$r_{013} = \frac{r_{01} + r_{13}e^{-2j\beta_1}}{1 + r_{01}r_{13}e^{-2j\beta_1}} \qquad (2)$$

où $r_{ij}$ est le coefficient de Fresnel à l'interface i-j (,j=0, 1 ou 3, « 0 » correspondant au milieu incident, « 1 » à la couche CA et « 3 » au milieu émergent) et $\beta_1 = 2\pi n_1 e_1 \cos\theta_1/\lambda$ est le facteur de phase associé à ladite couche, $\theta_1$ étant l'angle de réfraction dans la couche. Dans un premier temps, on considère une couche transparente d'indice réel $n_1$, la généralisation au cas d'une couche en matériau absorbant sera traitée plus loin. Toujours dans un premier temps, on considère une incidence qui peut ne pas être normale.

Les coefficients de Fresnel pour les polarisations « p » (TM) et « s » (TE) sont :

$$r_{ij}^{(p)} = \frac{\left(n_j \cos\theta_i - n_i \cos\theta_j\right)}{\left(n_j \cos\theta_i + n_i \cos\theta_j\right)}$$

et

$$r_{ij}^{(s)} = \frac{\left(n_i \cos\theta_i - n_j \cos\theta_j\right)}{\left(n_i \cos\theta_i + n_j \cos\theta_j\right)}$$

La condition antireflet correspond à $r_{013} = 0$ ce qui, dans le cas de milieux transparents (indices réels) donne deux familles de solutions :

5

- les couches dites « λ/2 », pour lesquelles $e_1 = \dfrac{m\lambda}{(2n_1 cos\theta_1)}$ où m est un entier, qui existent seulement si $n_0=n_3$ ; et

- les couches dites « λ/4 », pour lesquelles $n_1 e_1 = (2p + 1)\dfrac{\lambda}{4}$ (p entier).

Dans le cas où le milieu 1 (couche CA) est absorbant, son indice de réfraction $N_1=n_1-jk_1$ est complexe ; l'angle de réfraction - que l'on indique alors par $\Theta_1$ - et le coefficient de phase - $B_1$ - sont également complexes. Dans ce cas, $r_{013}=0$ impose : $r_{01,s}r_{13,p}= r_{01,p}r_{13,s}$ ; cette égalité ne peut être vraie que si une des trois conditions suivantes : $\Theta_1=0$ (incidence normale), $N_1{}^2=n_0{}^2$ (pas de couche) ou $n_0{}^2=n_3{}^2$ (milieux incident et émergent identiques) est satisfaite. Par conséquent, dans le cas de milieux extrêmes quelconques, la condition antireflet ne peut être satisfaite qu'en incidence normale. Sachant que $r_{011,p}=-r_{01,s}$ et que $r_{13,p}=-r_{13,s}$, l'équation (2) devient :

$$N_1{}^2 - j\frac{(n_3-n_0)}{tanB_1}N_1 - n_0n_3 = 0 \qquad (3)$$

L'équation (3) est transcendante et n'admet pas de solution analytique. Toutefois, on peut trouver des solutions correspondant aux cas extrêmes : couche fortement absorbante et couche faiblement absorbante.
Dans le cas fortement absorbant, on peut supposer que $e_1$ « λ car la lumière ne se propagerait pas à travers une couche très absorbante et épaisse ; par conséquent, $|B_1|<<1$ et on peut alors écrire, au second ordre en $B_1$ :

$tanB_1 \cong B_1 = \sqrt{n_3/n_0}\left(N_1/\sqrt{n_0n_3}\right)\delta_1$, où $\delta_1 = (2\pi n_0/\lambda)e_1$. Il est utile de séparer les parties réelles et imaginaires de l'équation, et d'utiliser les variables « réduites » $v_1 = n_1/\sqrt{n_0n_3}$ and $\kappa_1 = k_1/\sqrt{n_0n_3}$. L'équation (3) peut alors s'écrire sous la forme du système suivant :

$$v_1{}^2 = 1 + \kappa_1{}^2 \qquad (4a)$$

$$\delta_1 = \frac{\left(\frac{n_0}{n_3}-1\right)}{2v_1\kappa_1} \qquad (4b)$$

Etant donné que $\delta_1$ doit être réel et positif, on a la condition $n_0>n_3$ (« géométrie inversée »). En prenant $n_0=1,52$ et $n_3=1,34$ - ce qui correspond au cas verre/eau couramment utilisé en biophotonique - on trouve une épaisseur $e_1 = (\lambda/2\pi)$ $(n_0 - n_3)/2n_1k_1$ de l'ordre du nanomètre, ce qui confirme l'hypothèse initiale. Il est intéressant - et inattendu - que l'équation (4a) tend à la condition d'indice classique quand $\kappa_1$ - et donc $k_1$ - tend vers zéro. Une comparaison avec des résultats numériques permet de vérifier que l'équation (4a), bien que dérivée dans l'hypothèse d'une couche fortement absorbante, est approximativement valable pour toute valeur de $k_1$. Par contre, la valeur de $e_1$ obtenue à partir de l'équation (4b) ne tend pas vers $\lambda/4n_1$ ; par conséquent, l'équation (4b) n'a pas une validité générale.
Dans le cas faiblement absorbant on pose $B_1=\pi/2-\varepsilon_1$ (où $\varepsilon_1$ est une variable complexe), ce qui implique :

$\varepsilon_1 = \pi/2 - \sqrt{\dfrac{n_3}{n_0}}(v_1 - jk_1)\delta_1$. On peut alors écrire, au second ordre en $\kappa_1$ :

$$v_1{}^2 = 1 + \frac{\pi}{2}\sqrt{\frac{n_3}{n_0}}\left(\frac{n_0}{n_3} - 1\right)\kappa_1 - 3\kappa_1{}^2 + o(\kappa_1{}^3) \qquad (5a)$$

$$\delta_1 \simeq \frac{\pi}{2}\sqrt{\frac{n_0}{n_3}}\frac{1}{v_1}\left\{1 - \frac{4}{\pi}\frac{\sqrt{n_0/n_3}}{(n_0/n_3-1)}\kappa_1 + \kappa_1{}^2 + o(\kappa_1{}^3)\right\} \qquad (5b)$$

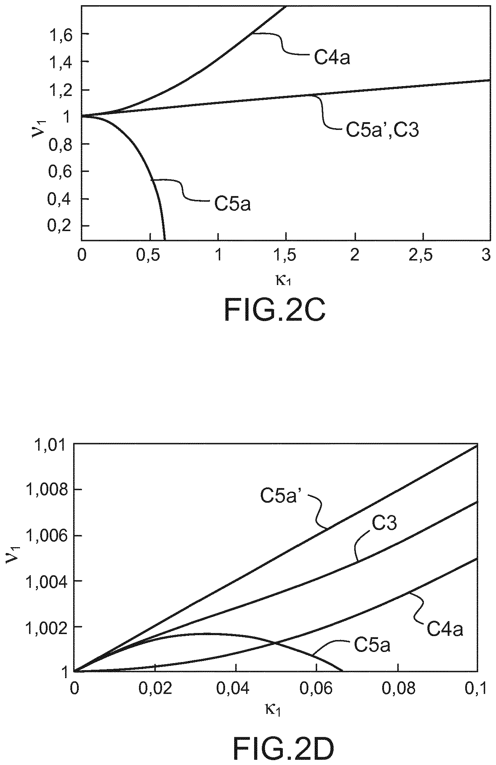

En pratique, l'équation (5a) - dont le domaine de validité s'avère très réduit - a peu d'intérêt car, comme mentionné plus haut, l'équation (4a) constitue une approximation satisfaisante pour toute valeur de $k_1$. Cela est illustré par la figure 2A, qui montre la relation $v_1(\kappa_1)$ ; les courbes correspondant à une solution numérique de l'équation (3) et à l'équation (4a) ne peuvent pas être distinguées. La figure 2B montre l'erreur - en pourcentage - de l'équation (4a) par rapport à la solution numérique : on peut voir que cette erreur est très faible. Les figures 2C et 2D sont des agrandissements de la figure 2A qui permettent d'étudier plus en détail le régime de faible absorption ; sur ces figures la courbe c4a correspond à l'équation (4a) valable pour une forte absorption, c3 à la solution numérique de l'équation (3), c5a à l'équation (5a) et c5a' à l'équation (5a) tronquée au premier ordre. On peut voir que l'équation (5a) et sa version au premier ordre constituent effectivement une meilleure approximation que l'équation 4a pour des faibles valeurs de $\kappa_1$, mais que l'équation (4a) demeure une assez bonne approximation dans tous les cas, tandis que l'équation (5a) perd rapidement toute pertinence.

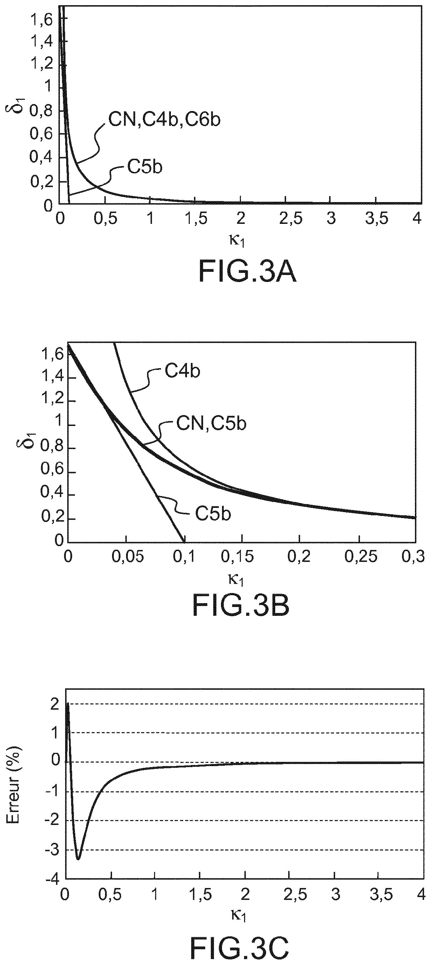

Les figures 3A et 3B illustrent la relation $\delta_1(\kappa_1)$ ; la courbe cN correspond à la solution numérique de l'équation 3, c4b correspond à l'équation 4b, valable pour $k_1$ grand, et c5b correspond à l'équation 5b. On peut voir que, dans ce cas, la solution obtenue pour $k_1$ élevé ne constitue pas une approximation acceptable pour $\kappa_1$ petit. Par contre, il existe une équation semi-empirique - correspondant à la courbe c6b - qui s'avère satisfaisante dans tous les cas. La figure 3C illustre l'erreur de cette solution semi-empirique par rapport à la solution numérique : elle n'excède jamais 3,5%. La solution semi-empirique est donnée par l'équation 6b ci-dessous ; l'équation 6a est simplement l'équation 4a qui, comme cela a été montré plus haut, peut être considérée générale et utilisée en remplacement de 5b même pour $\kappa_1$ petit :

$$v_1{}^2 = 1 + \kappa_1{}^2 \tag{6a}$$

$$\delta_1 \cong \frac{(n_0/n_3 - 1)}{2v_1\kappa_1}\left[1 - e^{-\frac{\kappa_1}{K}}\right] \tag{6b}.$$

où

$$K = \left\{[\pi/(n_0/n_3 - 1)]\sqrt{n_0/n_3}\right\}^{-1}$$

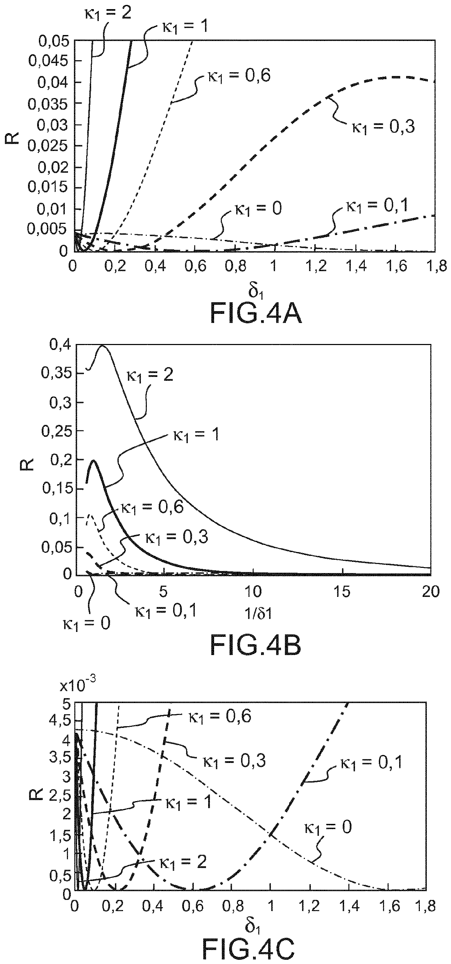

La figure 4A montre les courbes de réflectance en fonction de $\delta_1$ pour différentes valeurs de $\kappa_1$ ; la figure 4B montre les courbes de réflectance en fonction de $1/\delta_1$. Comme $1/\delta_1$ est proportionnel à $\lambda$, la figure 4B illustre comment la réflectance d'un substrat donné varie en fonction de la longueur d'onde d'éclairage. On peut remarquer qu'une couche antireflet absorbante dimensionnée pour opérer à une longueur d'onde $\lambda$ atténue la réflexion également à des longueurs d'onde $\lambda' > \lambda$. Cela permet d'utiliser ces supports également en éclairage polychromatique ; dans ce dernier cas, il convient d'effectuer le dimensionnement de la couche antireflet absorbante par rapport à la plus petite longueur d'onde utilisée pour l'éclairage.

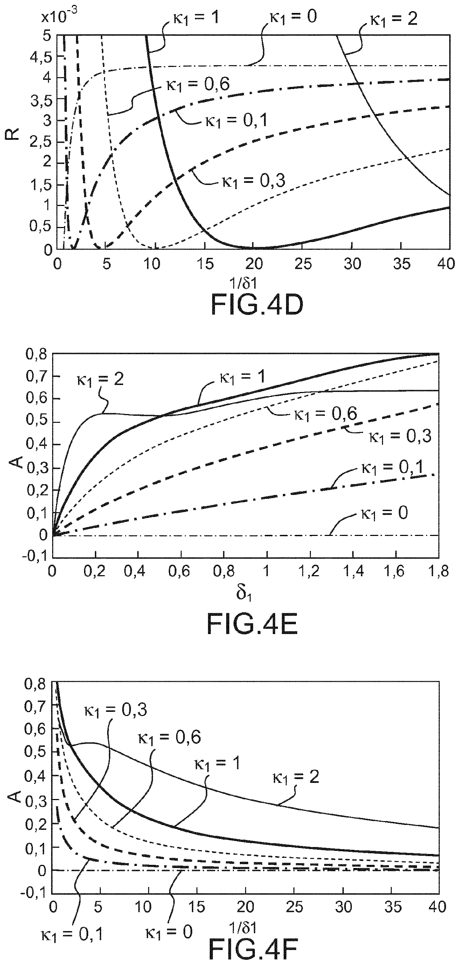

Les figures 4C et 4D sont des agrandissements des figures 4A et 4B, respectivement, montrant plus spécifiquement la région des faibles réflectances. Les figures 4E et 4F montrent les courbes d'absorbance pour différentes valeurs de $\kappa_1$ respectivement en fonction de $\delta_1$ et $1/\delta_1$.

Les figures 4A à 4D montrent que l'épaisseur réduite $\delta_1$ d'une couche antireflet absorbante est d'autant plus faible que la partie imaginaire réduite $\kappa_1$ de son indice de réfraction est important. En d'autres termes, plus la couche est absorbante, plus elle doit être mince. Les courbes 4E et 4F permettent de vérifier que l'absorbance à l'épaisseur $\delta_1$ donnée par l'équation 6b est pratiquement indépendante de $\kappa_1$ et vaut environ 0,1.

Dans leur article précité, G. Moiseev et S. V. Vinogradov ont étudié une couche antireflet absorbante utilisée en configuration non inversée (éclairage provenant du milieu de plus faible indice) ; ils ont trouvé une épaisseur d'autant plus grande que la partie imaginaire de l'indice de réfraction de la couche est élevée, conduisant à une absorbance qui augmente rapidement avec cette dernière. Dans ces conditions, la couche ANtiReflet ne peut exister que pour des valeurs très faibles de $k_1$ il ne serait pas possible d'utiliser une couche antireflet très absorbante en tant que couche amplificatrice de contraste. Ce problème ne se pose pas dans le cas considéré ici.

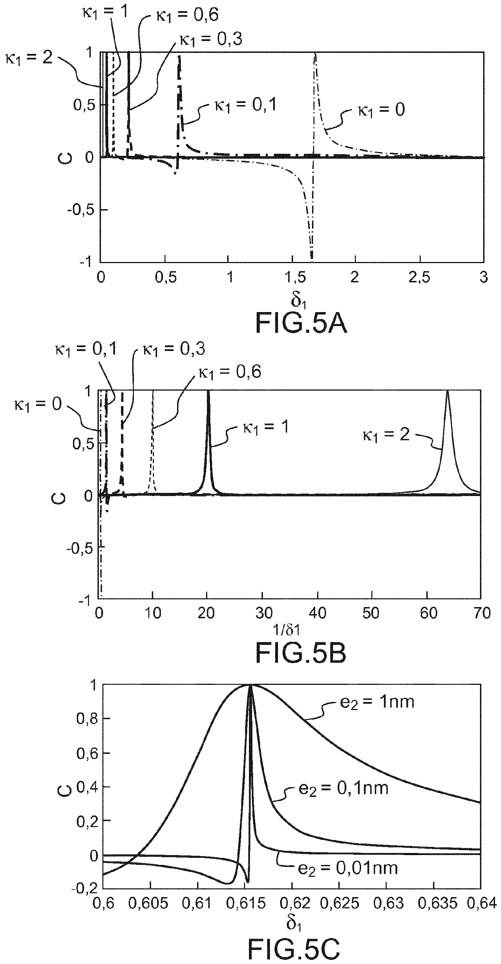

Les figures 5A à 5D permettent d'étudier le contraste avec lequel un échantillon peut être observé grâce à des supports comprenant une couche antireflet absorbante telle que décrite ci-dessus. On considère un substrat en verre, un milieu émergent constitué par de l'eau ($n_0/n_3 = 1,14$) et un échantillon constitué par une couche transparente d'indice de réfraction $n_2 = 1,46$. La figure 5A montre la valeur du contraste C avec lequel un échantillon d'épaisseur $e_2 = 1$ nm est observé, en fonction de l'épaisseur réduite $\delta_1$ pour les mêmes valeurs de $\kappa_1$ que celles considérées dans les figures 4A - 4F : $\kappa_1 = 0$ (couche antireflet non absorbante, ne faisant pas partie de l'invention) ; 0,1 ; 0,3 ; 0,6 ; 1 et 2. La figure 5B montre la valeur de ce contraste en fonction de $1/\delta_1$.

On remarque que :

- seule la couche non absorbante permet une véritable inversion du contraste (échantillon sombre sur fond clair) ; la couche $\kappa_1$=0,1 permet une telle inversion mais seulement à un niveau de contraste très faible ;
- la largeur des pics de contraste est d'autant plus faible - et donc la tolérance sur l'épaisseur réduite de la couche antireflet - que $\kappa_1$ est élevée. Dans le cas d'un objet à observer ayant une épaisseur de 1nm, pour $\kappa_1$=0,1 le contraste reste acceptable (0,4) même lorsque $\delta_1$ s'écarte de $\pm$10% de sa valeur optimale, mais cette tolérance peut difficilement dépasser 1% pour $\kappa_1$=1.

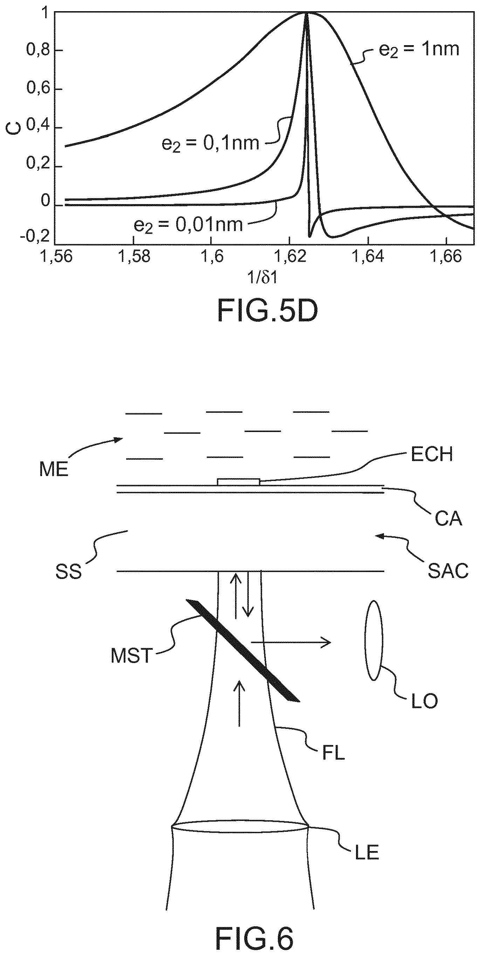

Les figures 5C et 5D permettent d'étudier l'influence de l'épaisseur de l'échantillon : elles montrent la valeur du contraste C en fonction de $\delta_1$ et de $1/\delta_1$ respectivement, dans le cas $\kappa_1$=0,1 et pour $e_2$=1 nm, 0,1 nm et 0,01 nm (il s'agit d'épaisseurs effectives d'échantillons pouvant être constitués d'atomes ou molécules éparses, disposés sur la surface de la couche amplificatrice de contraste). On remarque que le contraste C peut toujours atteindre une valeur de 1, mais que la tolérance sur $\delta_1$ est d'autant plus réduite que l'épaisseur $e_2$ est faible. Concrètement, comme $\delta_1$ dépend tant de l'épaisseur de la couche antireflet absorbante que de la longueur d'onde d'éclairage, dans le cas d'échantillons très fins il pourra être avantageux d'ajuster finement cette longueur d'onde pour maximiser le contraste.

Il peut être aussi avantageux de choisir une longueur d'onde d'éclairage et/ou une épaisseur de la couche antireflet absorbante telle que $\delta_1$ soit légèrement supérieure à sa valeur optimale, afin que le contraste devienne une fonction monotone de l'épaisseur de l'objet, ce qui en permet la cartographie.

La figure 6 représente un support amplificateur de contraste SAC comprenant un substrat transparent SS - par exemple en verre - servant de milieu incident, une couche antireflet en matériau absorbant CA déposée sur le dit substrat et en contact avec un milieu émergent ME, par exemple une solution aqueuse ou l'air. Un échantillon ECH est déposé sur une portion de la couche CE, du côté du milieu émergent. Le substrat est éclairé en incidence normale par un faisceau lumineux FL qui est, dans l'exemple considéré ici, un faisceau laser à profil gaussien, focalisé par une lentille LE au niveau de la couche antireflet. On sait en effet que, dans sa région focale (« beam waist »), un faisceau gaussien présente un front de phase plan, et peut donc être assimilé localement à une onde plane (cas considéré dans les développements théoriques qui précèdent). Un miroir semi-transparent MST dévie une portion de la lumière réfléchie par l'ensemble substrat SS/couche CA/échantillon ECH/milieu émergent ME, pour la diriger vers un objectif LO, permettant l'observation dudit échantillon. L'observation peut se faire par balayage ou « plein champ ». En variante, il est été possible d'utiliser un faisceau de lumière parallèle ou un système de vision télécentrique. Il convient de noter que la cohérence spatiale de la lumière incidente et son état de polarisation n'ont pas d'importance. En revanche, si on veut observer un contraste d'intensité, il convient d'utiliser un éclairage à bande étroite ; un éclairage polychromatique conduit à un contraste qui n'est pas tant d'intensité que de couleur (échantillon observé avec une couleur différente de celle du fond et couleurs différentes selon l'épaisseur de l'échantillon).

Dans le montage de la figure 6, les lentilles LO et LE sont interchangeables. Par ailleurs, la réflexion parasite sur la face avant du substrat peut être utilement atténuée par des techniques telles que : immersion dans une huile, l'existence d'un biseau entre la face avant et la face arrière, un filtrage spatial, un traitement antireflet classique.

Pour concevoir un support amplificateur de contraste du type illustré sur la figure 6 on peut procéder de la façon suivante :

- Premièrement, on détermine la longueur d'onde d'éclairage (ou la plus petite longueur d'onde d'éclairage, si ce dernier est polychromatique) λ, en fonction de l'application considérée ou de différentes contraintes technologiques.
- Ensuite, on choisit un premier matériau destiné à constituer le substrat et un matériau destiné à constituer le « milieu ambiant » ou « émergent ». Souvent, le choix du milieu ambiant est déterminé par l'application considérée (généralement une solution aqueuse pour les applications biologiques) ; le choix du matériau constituant le substrat est dicté par des considérations technologiques et par la contrainte $n_3 < n_0$ à la longueur d'onde λ. Souvent, on choisira un substrat en verre, et un milieu ambiant constitué par l'air (rapport $n_3/n_0$ compris entre 1,45 et 1,7) ou l'eau (rapport $n_3/n_0$ compris entre 1,1 et 1,3).
- Puis, l'équation 6a est utilisée pour déterminer la relation liant la partie réelle et la partie imaginaire de l'indice de réfraction du matériau constituant la couche antireflet absorbante. Un matériau satisfaisant cette relation est alors choisi - ou conçu. Par exemple, on peut choisir un matériau de départ transparent en fonction de considérations technologiques diverses - par exemple un polymère ; prendre la partie réelle de son indice de réfraction comme une donnée imposée ; et modifier la partie imaginaire dudit indice de réfraction par l'ajout d'impuretés (colorants, nanoparticules...) pour que l'équation 6a soit satisfaite.
- Enfin, l'épaisseur de ladite couche est déterminée en appliquant l'équation 6b (ou l'une des équations 4b ou 5b, qui en constituent des cas particuliers).

On procède ensuite à la fabrication du support, par des techniques conventionnelles, telles que le revêtement à la tournette, par immersion, au rouleau, par sédimentation ou par évaporation ; le dépôt chimique ou physique en phase vapeur, l'implantation ionique, le dépôt électrolytique, etc.

La couche antireflet absorbante peut être métallique (et notamment en or), semi-conductrice, conductrice non-métallique, en polymère contenant des pigments ou colorants, en matériau inorganique (minéral) contenant des centres colorés, etc. Parmi les matériaux semi-conducteurs convenant à la réalisation de couches antireflet absorbantes on peut mentionner : le germanium (pour des applications dans l'ultraviolet (UV) proche, par exemple à 354 nm), le $TiO_2$ (également dans l'UV proche), le siliciure de molybdène, de nickel ou de titane (dans le visible), le siliciure de tungstène (dans le proche infrarouge ou dans le proche UV), le siliciure de zirconium (dans le visible ou le proche UV) le tantale ou le vanadium (dans le visible), etc. Elle peut également contenir des nanoparticules métalliques. Elle peut être magnétique, ce qui présente un intérêt pour l'étude d'échantillons qui sont à leur tour magnétiques. L'utilisation de couches conductrices - métalliques ou pas - permet d'appliquer une différence de potentiel contrôlée à l'échantillon et/ou de réaliser une « imagerie électrochimique » permettant d'étudier des phénomènes d'électrodépôt, corrosion, catalyse, etc. Une variante particulièrement intéressante consiste à réaliser un support monolithique, dans lequel la couche antireflet absorbante est une couche d'impuretés implantées - par exemple par implantation ionique - à la surface du substrat ; un tel substrat peut être nettoyé et réutilisé, sans danger d'abimer la couche. Une couche antireflet « absorbante » ne doit pas nécessairement être absorbante au sens propre : en variante, il peut s'agir d'une couche diffusante, la diffusion « imitant » l'absorption et pouvant également être modélisée par un indice de réfraction complexe.

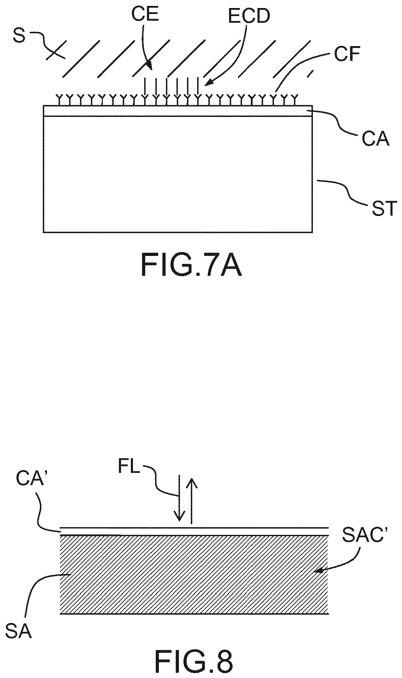

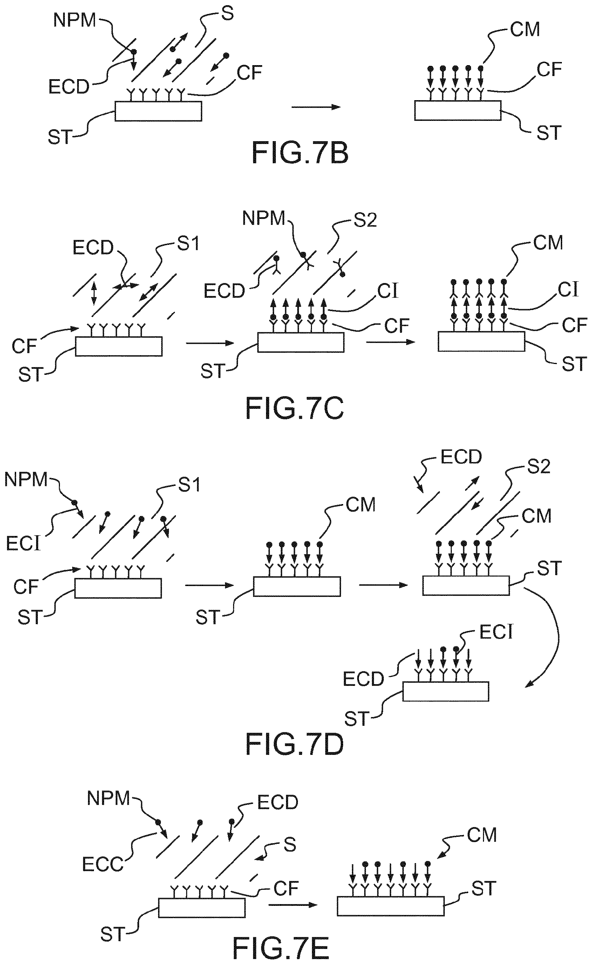

Un substrat amplificateur de contraste tel que décrit ci-dessus permet également la réalisation de biopuces pour la détection et/ou le dosage d'espèces chimiques ou biologique. Par exemple, comme illustré sur la figure 7, il est possible de déposer une couche fonctionnalisée CF sur la couche amplificatrice de contraste CA. Cette couche fonctionnalisée est mise en contact avec une solution S, par exemple aqueuse, contenant l'espèce chimique ou biologique à détecter ECD. Cette dernière est fixée par la couche fonctionnalisée et forme une couche mince supplémentaire CE, constituant l'échantillon à observer. En pratique, dans le cas d'une biopuce, on déposera plusieurs plots fonctionnalisés différents, permettant de fixer sélectivement des espèces chimiques ou biologiques différentes. En observant la biopuce au microscope, dans les conditions décrites ci-dessus, on peut identifier facilement les espèces effectivement présentes dans la solution. Dans certains modes de réalisation, une même couche peut réaliser à la fois la fonction chimique de fixation sélective et la fonction optique d'amplification du contraste.

De préférence, en dehors des plots on peut prévoir une couche de passivation empêchant la fixation de toute espèce chimique ou biologique contenue dans ladite solution (« passivation chimique »). On peut utiliser par exemple un polyéthylène glycol, un polymère fluoré, ou un alkyl fluoré, par exemple fonctionnalisés par des thiols dans le cas de l'or. Cette couche de passivation peut être déposée en phase vapeur ou en phase liquide après la fabrication des plots. En variante ou en complément on peut utiliser une couche antireflet absorbante discontinue, présente (ou présentant une épaisseur optimale) seulement en correspondance des plots ; on parle alors de « passivation optique ».

Lorsqu'on souhaite détecter ou déposer des espèces chimiques ou biologiques, il est également possible d'utiliser un substrat uniquement pourvu de la couche fonctionnalisée CF. Dans ce cas, la couche antireflet absorbante est constituée par les espèces fixées par ladite couche CF.

Selon un premier mode de réalisation, illustré sur la figure 7B, la couche fonctionnalisée est mise en contact avec une solution contenant une espèce chimique ou biologique ECD à détecter ou doser, marquée par des nanoparticules métalliques NPM et susceptible de se fixer sur ladite couche fonctionnalisée de manière à former une couche métallique CM. Cette couche peut être en réalité discontinue, mais elle apparait continue à l'échelle de la longueur d'onde de la lumière visible (plusieurs centaines de nanomètres), avec une épaisseur effective qui peut être une fraction du diamètre de la nanoparticule, et avec un indice de réfraction effectif. L'observation se fait de la manière décrite plus haut, la couche métallique ainsi constituée servant à la fois de couche amplificatrice de contraste et d'échantillon. Pour un temps de contact déterminé entre la solution et la couche fonctionnalisée, l'épaisseur de la couche métallique dépend de la teneur en espèce chimique ou biologique, ce qui permet de réaliser un dosage.

En variante, les nanoparticules métalliques peuvent être remplacées par un marqueur absorbant, par exemple une molécule fluorescente (à noter que la fluorescence, en soi, n'est pas exploitée, mais une molécule fluorescente est fortement absorbante).

L'inconvénient du premier mode de réalisation est de ne permettre que la détection d'une espèce chimique ou biologique marquée. Les modes de réalisation suivants ne présentent pas cet inconvénient.

Selon le deuxième mode de réalisation (figure 7C), la couche fonctionnalisée est placée en contact avec une première solution S1 contenant l'espèce chimique ou biologique à détecter ou doser, de manière à former une couche dite intermédiaire CI. Cette couche intermédiaire n'est pas observable. Pour la révéler, on la met en contact avec une deuxième solution S2, contenant une espèce chimique ou biologique dite auxiliaire ECA, marquée par des nanoparticules métalliques (ou un marqueur absorbant) et susceptible de se fixer sur ladite couche intermédiaire pour former la couche métallique (ou absorbante) CM.

La technique peut être quantitative si l'espèce à détecter est présente en quantité insuffisante pour saturer la couche fonctionnalisée et, par contre, l'espèce auxiliaire est présente en excès. Dans ce cas, en effet, l'épaisseur et l'indice effectifs de la couche CM - et donc l'intensité du signal lumineux observé - dépendrons de la concentration de l'espèce à détecter.

Ce deuxième mode de réalisation ne peut être utilisé que si l'espèce chimique ou biologique à détecter présente au moins deux sites actifs, il ne s'applique donc pas, par exemple, aux haptènes. En outre, il est assez complexe à mettre en œuvre.

Les modes de réalisation suivants ne présentent pas cet inconvénient.

Selon le troisième mode de réalisation (figure 7D), la couche fonctionnalisée est placée en contact avec une première solution (S1) contenant une espèce chimique ou biologique, dite espèce intermédiaire ECI, marquée par des nanoparticules métalliques ou un marqueur absorbant et susceptible de se fixer sur ladite couche de fonctionnalisation pour former ladite couche métallique ou absorbante (CM) continue ou discontinue. Ensuite, l'ensemble ainsi obtenu est mis en contact avec une deuxième solution (S2) contenant l'espèce chimique ou biologique à détecter ou doser, laquelle présente une affinité avec ladite couche de fonctionnalisation supérieure à celle de ladite espèce intermédiaire. Ainsi, l'espèce intermédiaire est déplacée et ladite couche métallique ou absorbante est supprimée au moins en partie, ce qui se traduit par une augmentation du signal lumineux. La technique s'applique à la fois à un niveau qualitatif à la détection et à un niveau quantitatif au dosage des espèces ciblées. Un avantage de cette approche est que ses deux étapes peuvent être dissociées : les supports peuvent être fournis avec la couche CM déjà formée, prêts à être utilisés comme capteurs chimiques ou biologiques.

Selon un quatrième mode de réalisation (figure 7E), l'on place ladite couche fonctionnalisée en contact avec une solution S contenant l'espèce chimique ou biologique à doser, ainsi que ladite espèce chimique ou biologique concurrente ECC, l'une des deux espèces (de préférence l'espèce concurrente) étant marquée par des nanoparticules métalliques ou un marqueur absorbant. Ainsi, l'on obtient une couche métallique ou absorbante CM dont l'épaisseur effective et l'indice effectif dépendent du rapport entre la concentration de ladite espèce chimique ou biologique concurrente et celle de la dite espèce chimique ou biologique à doser. Comme dans les autres modes de réalisation, le signal dépend de cette épaisseur effective et de cet indice effectif.

Les espèces chimiques ou biologiques peuvent être, par exemple, anticorps, antigènes, protéines, ADN, ARN, saccharides, enzymes, ions métalliques (notamment pour des applications au contrôle des eaux), molécules aromatiques, molécules organiques telles que des hydrocarbures, microorganismes, etc.

Au lieu d'être métallique ou absorbant, le marqueur peut être diffusant. En effet, comme cela a été expliqué plus haut, l'effet de la diffusion peut être exprimé par un indice de réfraction ayant une partie imaginaire. Ainsi, des nanoparticules diélectriques telles que des nanoparticules minérales de silice ou d'alumine, des dendrimères, des nanoparticules de latex, des vésicules, ou des virus peuvent jouer le même rôle que les nanoparticules métalliques.

Les techniques de détection ou dosage décrites ci-dessus s'appliquent également lorsque la couche fonctionnalisée est déposée sur une couche d'amplification de contraste telle que décrite plus haut. La couche fonctionnalisée, et le cas échéant la couche d'amplification de contraste, peuvent être structurées en plots, et la surface en dehors de ces plots peut être passivée chimiquement et/ou optiquement, comme expliqué plus haut.

Jusqu'ici on a considéré uniquement le cas où l'éclairage et l'observation se font à travers d'un substrat présentant un indice de réfraction (réel) supérieur à celui du milieu ambiant - ce qu'on appelle une « géométrie inversée » ou « face arrière ». En variante, il est également possible d'opérer en configuration « face avant », c'est-à-dire en effectuant l'éclairage et l'observation à travers du milieu ambiant ; dans ce cas, le substrat doit présenter un indice de réfraction plus faible que celui dudit milieu ambiant : $n_0 < n_3$.

Une autre généralisation consiste à considérer un milieu incident et/ou un milieu émergent absorbant. Le cas le plus intéressant est celui où le milieu incident est transparent et le milieu émergent absorbant : en effet, si le milieu incident était fortement absorbant, la lumière ne pourrait pas s'y propager pour parvenir à la couche antireflet.

En partant de l'équation (3), dans le cas $\kappa_1 > 0,15$ et en remplaçant $n_3$ par $N_3 = n_3 - jk_3$ on obtient :

$$\nu_1{}^2 - \kappa_1{}^2 = 1 + \sqrt{\frac{n_0}{n_3}}\,\kappa_3\, \frac{2\nu_1\kappa_1 - \sqrt{\frac{n_0}{n_3}}\kappa_3}{\left(\frac{n_0}{n_3}-1\right)} \qquad (7a)$$

$$\delta_1 = \frac{\left(\frac{n_0}{n_3}-1\right)}{2\nu_1\kappa_1 - \sqrt{\frac{n_0}{n_3}}\kappa_3} \qquad (7b)$$

où

$$\kappa_3 = k_3 / \sqrt{n_0 n_3}.$$

On remarque que, dans l'équation 7b, une valeur élevée de $\kappa_3$ peut inverser le signe de $\delta_1$. Par conséquent, on peut réaliser une couche antireflet absorbante déposée sur un substrat qui est à son tour absorbant, éclairée par sa face opposée audit substrat (configuration « face avant »). Il peut s'agir par exemple d'une couche métallique déposée sur un substrat semiconducteur ou l'inverse, ce qui a des applications par exemple en microélectronique. Des couches de ce type ont été décrites par l'article précité de R. M. A. Azzam et al. L'article précité de M. A. Kats et al., en outre, a décrit des couches semblables mais n'annulant pas totalement la réflexion. Ces publications, cependant, ne fournissent aucune méthode générale et systématique de conception de telles couches.

La théorie exposée ci-dessus permet de concevoir et fabriquer un support amplificateur de contraste comprenant une couche en matériau absorbant déposée sur un substrat absorbant comme cela a été décrit plus haut pour le cas d'un substrat transparent - mais en utilisant les équations 7a/7b au lieu des équations 6a/6b.

Un support amplificateur de contraste SAC' de ce type, comprenant un substrat absorbant SA et une couche antireflet absorbante CA' décrite par les équations 7a et 7b, est représenté sur la figure 8. L'observation se fait « face avant » (du côté de la couche opposé au substrat) au moyen d'un faisceau de lumière parallèle FL, ou d'un faisceau laser gaussien focalisé comme dans le cas de la figure 6. Les matériaux mentionnés en référence à la couche CA peuvent également être utilisés pour la réalisation d'une couche CA'. Cette dernière peut également être fonctionnalisée ou être réalisée par des espèces chimiques ou biologiques, éventuellement marquées, fixées par une couche de fonctionnalisation, en particulier pour des applications de détection ou dosage.

## Revendications

1. Support amplificateur de contraste (SAC) pour l'observation d'un échantillon déposé sur ledit support, comprenant un substrat transparent (MI, SS) portant une couche en matériau absorbant (CA) dont l'indice de réfraction complexe $N_1 = n_1 - jk_1$ et l'épaisseur sont choisis de telle sorte que ladite couche se comporte en tant que couche antireflet lorsqu'elle est éclairée en incidence normale à une longueur d'onde d'éclairage $\lambda$ à travers ledit substrat, la face de ladite couche opposée audit substrat étant en contact avec un milieu dit ambiant (ME) transparent dont l'indice de réfraction $n_3$ est inférieur à celui de l'indice de réfraction $n_0$ dudit substrat et dans lequel, à ladite longueur d'onde d'éclairage $\lambda$, l'indice de réfraction $n_0$ du substrat, les parties réelles et imaginaires de l'indice de réfraction complexe de la couche $N_1 = n_1 - jk_1$ et l'épaisseur $e_1$ de la couche satisfont aux conditions suivantes :

   a)

   $$\nu_1{}^2 = 1 + \kappa_1{}^2 \, ;$$

   b)

   $$\delta_1 = \frac{(n_0/n_3 - 1)}{2\nu_1 \kappa_1} \left[ 1 - e^{-\frac{\kappa_1}{K}} \right] \qquad ;$$

   et

   c) $k_1 \geq 0{,}01$, et de préférence $k_1 \geq 0{,}1$ où :

   $$\delta_1 = \frac{2\pi n_0}{\lambda} e_1 ;$$

$$\nu_1 = \frac{n_1}{\sqrt{n_0 n_3}} \; ;$$

-

$$\kappa_1 = \frac{k_1}{\sqrt{n_0 n_3}} \; ;$$

et

-

$$K = \left\{ [\pi/(n_0/n_3 - 1)] \sqrt{n_0/n_3} \right\}^{-1}$$

avec une tolérance inférieure ou égale à 5%, et de préférence inférieure ou égale à 0,3%, pour $n_1$ et $k_1$, et avec une tolérance inférieure ou égale à 30% et de préférence inférieure ou égale à 5% pour $e_1$.

**2.** Support amplificateur de contraste selon la revendication 1 dans lequel l'indice de réfraction du substrat $n_0$, l'indice de réfraction complexe de la couche $N_1 = n_1 - jk_1$ et l'épaisseur $e_1$ de la couche satisfont, en outre, avec lesdites tolérances et pour ladite longueur d'onde d'éclairage $\lambda$, aux conditions suivantes :

a')

$$\nu_1{}^2 = 1 + \kappa_1{}^2 \; ;$$

b')

$$\delta_1 = \frac{n_0/n_3 - 1}{2\nu_1 \kappa_1} \; ;$$

et
c') $k_1 \geq 0,15$.
où :

-

$$\delta_1 = \frac{2\pi n_0}{\lambda} e_1 \; ;$$

-

$$\nu_1 = \frac{n_1}{\sqrt{n_0 n_3}} \; ;$$

-

$$\kappa_1 = \frac{k_1}{\sqrt{n_0 n_3}} \; ;$$

et

-

$$K = \left\{ [\pi / (n_0/n_3 - 1)] \sqrt{n_0/n_3} \right\}^{-1} .$$

3. Support amplificateur de contraste selon l'une des revendications précédentes dans lequel ledit substrat est en verre.

4. Support amplificateur de contraste selon la revendication 3 dans lequel le rapport $n_0/n_3$ est choisi compris entre 1,1 et 1,3.

5. Support amplificateur de contraste selon la revendication 3 dans lequel le rapport $n_0/n_3$ est choisi compris entre 1,45 et 1,7.

6. Support amplificateur de contraste selon l'une des revendications précédentes dans lequel ladite couche en matériau absorbant est choisie parmi :

   - une couche d'impuretés implantées dans ledit substrat ;
   - une couche métallique ;
   - une couche semi-conductrice;
   - un alliage composite metal/semi-conducteur
   - une couche en matériau absorbant magnétique.
   - une couche de nanoparticules métalliques;
   - une couche conductrice non métallique ;
   - une couche diffusante;
   - une couche de polymère ou de photoresist contenant des pigments ou colorants ;
   - une couche diélectrique minérale contenant des centres colorés ;
   - une couche hybride composite comprenant une phase continue dans laquelle sont dispersées des nanoparticules ; et
   - une couche de graphène ou une couche de graphène fonctionnalisée.

7. Support amplificateur de contraste selon l'une des revendications précédentes dans lequel ladite couche en matériau absorbant comprend une couche fonctionnalisée, susceptible de fixer au moins une espèce chimique ou biologique.

8. Procédé de fabrication d'un support amplificateur de contraste pour l'observation d'un échantillon déposé sur ledit support, comprenant un substrat transparent (MI, S) portant une couche en matériau absorbant (CA), ledit procédé comprenant une phase de conception dudit support et une phase de fabrication matérielle du support ainsi conçu ; dans lequel ladite étape de conception comprend les étapes suivantes :

   i) choisir une longueur d'onde d'éclairage $\lambda$ ;
   ii) choisir un matériau constituant ledit substrat et présentant, à ladite longueur d'onde d'éclairage $\lambda$, un indice de réfraction réel $n_0$ ;
   iii) choisir un milieu ambiant (ME) en contact avec ladite couche du côté opposé audit substrat et présentant, à ladite longueur d'onde d'éclairage $\lambda$, un indice de réfraction réel $n_3 < n_0$ ;
   iv) déterminer un indice de réfraction complexe nominal $N_1 = n_1 - jk_1$ et une épaisseur nominale $e_1$ de ladite couche telles qu'elle se comporte en tant que couche antireflet lorsqu'elle est éclairée en incidence normale à ladite longueur d'onde d'éclairage $\lambda$ à travers ledit substrat, la face de ladite couche opposée audit substrat étant en contact avec ledit milieu ambiant ; et
   v) choisir un matériau constituant ladite couche en matériau absorbant et présentant, à ladite longueur d'onde d'éclairage $\lambda$, un indice de réfraction complexe dont les parties réelle et imaginaire coïncident avec celles dudit indice de réfraction complexe nominal à moins d'une tolérance inférieure ou égale à 5%, et de préférence inférieure ou égale à 0,3% dans lequel, lors de ladite étape iv), ledit indice de réfraction complexe nominal et ladite épaisseur nominale sont choisis satisfaisant aux conditions suivantes :

   a)

$$\nu_1{}^2 = 1 + \kappa_1{}^2 ;$$

   b)

$$\delta_1 = \frac{(n_0/n_3 - 1)}{2\nu_1\kappa_1}\left[1 - e^{-\frac{\kappa_1}{K}}\right]$$

; et

c) $k_1 \geq 0{,}01$, et de préférence $k_1 \geq 0{,}1$

où :

- 

$$\delta_1 = \frac{2\pi n_0}{\lambda} e_1;$$

- 

$$\nu_1 = \frac{n_1}{\sqrt{n_0 n_3}} \; ;$$

- 

$$\kappa_1 = \frac{k_1}{\sqrt{n_0 n_3}} \; ; \text{et}$$

et

- 

$$K = \left\{[\pi/(n_0/n_3 - 1)]\sqrt{n_0/n_3}\right\}^{-1}$$

9. Procédé selon la revendication 8 dans lequel l'indice de réfraction complexe nominal et l'épaisseur nominal de la couche sont déterminés de manière à satisfaire, pour ladite longueur d'onde d'éclairage λ, aux conditions suivantes :

a')

$$\nu_1{}^2 = 1 + \kappa_1{}^2$$

b')

$$\delta_1 = \frac{n_0/n_3 - 1}{2\nu_1\kappa_1};$$

et

c') $k_1 \geq 0{,}15$.

où :

- 

$$\delta_1 = \frac{2\pi n_0}{\lambda} e_1;$$

-

$$\nu_1 = \frac{n_1}{\sqrt{n_0 n_3}} \; ;$$

$$\kappa_1 = \frac{k_1}{\sqrt{n_0 n_3}} \; ;$$

et

$$K = \left\{ \left[ \pi / (n_0/n_3 - 1) \right] \sqrt{n_0/n_3} \right\}^{-1} .$$

**10.** Procédé d'observation d'un échantillon comportant les étapes suivantes :

A. Disposer ledit échantillon sur une couche en matériau absorbant (CA) d'épaisseur $e_1$ présentant un indice de réfraction complexe $N_1 = n_1 - jk_1$, comprise entre un premier milieu transparent, dit incident (MI, SS), ayant un indice de réfraction réel $n_0$ et un deuxième milieu transparent, dit émergent (ME), ayant un indice de réfraction réel $n_3 < n_0$ ;
B. éclairer ledit échantillon en incidence normale au moins à ladite longueur d'onde d'éclairage $\lambda$ à travers ledit milieu incident ;
C. observer l'échantillon ainsi éclairé, également à travers-ledit milieu incident ;

dans lequel ladite couche en matériau absorbant présente un indice de réfraction complexe et une épaisseur tels que :

a)

$$\nu_1{}^2 = 1 + \kappa_1{}^2 \; ;$$

b)

$$\delta_1 = \frac{(n_0/n_3 - 1)}{2\nu_1 \kappa_1} \left[ 1 - e^{-\frac{\kappa_1}{K}} \right]$$

; et
c) $k_1 \geq 0,01$, et de préférence $k_1 \geq 0,1$
où :

$$\delta_1 = \frac{2\pi n_0}{\lambda} e_1 ;$$

$$\nu_1 = \frac{n_1}{\sqrt{n_0 n_3}} \; ;$$

$$\kappa_1 = \frac{k_1}{\sqrt{n_0 n_3}} \ ;$$

et

-

$$K = \left\{ \left[ \pi/(n_0/n_3 - 1) \right] \sqrt{n_0/n_3} \right\}^{-1}$$

avec une tolérance inférieure ou égale à 5%, et de préférence inférieure ou égale à 0,3%, pour $n_0$, $n_1$ et $k_1$ et avec une tolérance inférieure ou égale à 30% et de préférence inférieure ou égale à 5% pour $e_1$.

11. Procédé selon la revendication 10 dans lequel ladite étape A est mise en œuvre au moyen d'un support amplificateur de contraste (SAC) selon l'une des revendications 1 à 8, dont le substrat constitue ledit milieu incident.

12. Procédé selon l'une des revendications 10 où 11 dans lequel ledit échantillon est éclairé au moins à ladite longueur d'onde d'éclairage λ par un faisceau (FL) de lumière parallèle ou focalisé de manière à former un cône d'éclairage présentant un demi-angle d'ouverture inférieure ou égale à 20°.

13. Procédé selon la revendication 11 dans lequel ledit échantillon est éclairé au moins à ladite longueur d'onde d'éclairage λ par un faisceau laser focalisé.

14. Procédé de détection ou dosage d'au moins une espèce chimique ou biologique comportant les étapes suivantes :

I. procurer un support amplificateur de contraste (SAC) selon la revendication 7, comprenant une couche ou surface fonctionnalisée (CF), susceptible de fixer au moins une espèce chimique ou biologique ;
II. placer ladite couche ou surface fonctionnalisée en contact avec au moins une solution (S0, S0', S1, S1', S2) contenant une espèce chimique ou biologique marquée par des nanoparticules métalliques (NPM) ou un marqueur absorbant ou diffusant, ladite espèce étant susceptible de se fixer sur ladite couche ou surface fonctionnalisée, soit directement, soit par l'intermédiaire d'une ou plusieurs autres espèces chimiques ou biologiques, moyennant quoi lesdites particules forment une couche métallique, absorbante ou diffusante continue ou discontinue ;
III. éclairer ledit support amplificateur de contraste en incidence normale au moins à ladite longueur d'onde d'éclairage λ à travers ledit substrat ;
IV. observer ledit support amplificateur de contraste ainsi éclairé, également à travers ledit substrat.

15. Procédé de détection ou dosage d'au moins une espèce chimique ou biologique comportant les étapes suivantes :

I. procurer un substrat transparent (SS) portant une couche ou surface fonctionnalisée (CF), susceptible de fixer au moins une espèce chimique ou biologique ;
II. placer ladite couche ou surface fonctionnalisée en contact avec au moins une solution (S0, S0', S1, S1', S2) contenant une espèce chimique ou biologique marquée par des nanoparticules métalliques (NPM) ou un marqueur absorbant ou diffusant, ladite espèce étant susceptible de se fixer sur ladite couche ou surface fonctionnalisée - soit directement, soit par l'intermédiaire d'une ou plusieurs autres espèces chimiques ou biologiques - pour former une couche métallique, absorbante ou diffusante continue ou discontinue, ledit substrat transparent formant, avec ladite couche ou surface fonctionnalisée (CF) et la couche métallique, absorbante ou diffusante ainsi formée, un support amplificateur de contraste selon l'une des revendications 1 à 5 ;
III. éclairer ledit support amplificateur de contraste en incidence normale au moins à ladite longueur d'onde d'éclairage λ à travers ledit substrat ;
IV. observer ledit support amplificateur de contraste ainsi éclairé, également à travers ledit substrat.

16. Procédé de détection ou dosage d'au moins une espèce chimique ou biologique selon la revendication 15, dans lequel ladite étape II comprend les sous-étapes consistant à :

II-1) Placer ladite couche ou surface fonctionnalisée en contact avec une première solution (S1) contenant l'espèce chimique ou biologique à détecter ou doser (ECD), de manière à former une couche dite intermédiaire (CI) ; et

II-2) Placer ladite couche intermédiaire en contact avec une deuxième solution (S2), contenant une espèce chimique ou biologique dite auxiliaire (ECA), marquée par des nanoparticules métalliques ou un marqueur absorbant ou diffusant et susceptible de se fixer sur ladite couche intermédiaire pour former ladite couche métallique, absorbante ou diffusante continue ou discontinue.

17. Procédé de détection ou dosage d'au moins une espèce chimique ou biologique selon la revendication 15, dans lequel ladite étape II comprend les sous-étapes consistant à :

II-1') Placer ladite couche ou surface fonctionnalisée en contact avec une première solution (S1') contenant une espèce chimique ou biologique, dite espèce intermédiaire (ECI), marquée par des nanoparticules métalliques ou un marqueur absorbant ou diffusant et susceptible de se fixer sur ladite couche de fonctionnalisation pour former ladite couche métallique, absorbante ou diffusante continue ou discontinue ; et

II-2') Placer ladite couche ou surface fonctionnalisée et ladite couche métallique, absorbante ou diffusante en contact avec une deuxième solution (S2) contenant ladite espèce chimique ou biologique à détecter ou doser, laquelle présente une affinité avec ladite couche ou surface fonctionnalisée supérieure à celle de ladite espèce intermédiaire, moyennant quoi ladite espèce intermédiaire est déplacée et ladite couche métallique, absorbante ou diffusante est supprimée au moins en partie.

18. Procédé de dosage d'au moins une espèce chimique ou biologique selon la revendication 15, dans lequel, lors de ladite étape II, on place ladite couche ou surface fonctionnalisée en contact avec une solution (S0') contenant l'espèce chimique ou biologique à doser (ECD), ainsi que ladite espèce chimique ou biologique concurrente (ECC), l'une des deux espèces étant marquée par des nanoparticules métalliques ou un marqueur absorbant ou diffusant, moyennant quoi on obtient une couche métallique, absorbante ou diffusante continue ou discontinue dont l'épaisseur effective dépend du rapport entre la concentration de ladite espèce chimique ou biologique concurrente et celle de la dite espèce chimique ou biologique à doser.

**Patentansprüche**

1. Kontrastverstärkungsträger (SAC) zum Beobachten einer auf den Träger abgesetzten Probe, mit einem transparenten Substrat (MI, SS), das eine Schicht aus absorbierendem Material (CA) umfasst, dessen komplexer Brechungsindex $N_1 = n_1 - jk_1$ und Dicke so gewählt sind, dass sich die Schicht als Antireflexionsschicht verhält, wenn sie bei normalem Einfall mit einer Beleuchtungswellenlänge $\lambda$ durch das Substrat beleuchtet wird, wobei die dem Substrat gegenüberliegende Fläche der Schicht in Kontakt mit einem transparenten Umgebungsmedium (ME) steht, dessen Brechungsindex $n_3$ kleiner als der Brechungsindex $n_0$ des Substrats ist, und wobei bei der Beleuchtungswellenlänge $\lambda$ der Brechungsindex $n_0$ des Substrats, der Real- und Imaginärteil des komplexen Brechungsindex der Schicht $N_1 = n_1 - jk_1$ und die Dicke $e_1$ der Schicht die folgenden Bedingungen erfüllen:

a)

$$\nu_1{}^2 = 1 + \kappa_1{}^2 \; ;$$

b)

$$\delta_1 = \frac{(n_0/n_1 - 1)}{2\nu_1\kappa_1}\left[1 - e^{-\frac{\kappa_1}{\kappa}}\right] \;;$$

und

c) $k_1 \geq 0{,}01$ und vorzugsweise $k_1 \geq 0{,}1$;

wobei

-

$$\delta_1 = \frac{2\pi n_0}{\lambda} e_1;$$

$$\nu_1 = \frac{n_1}{\sqrt{n_0 n_3}} \ ;$$

$$\kappa_1 = \frac{k_1}{\sqrt{n_0 n_3}} \ ;$$

und

$$K = \left\{ [\pi/(n_0/n_3 - 1)]\sqrt{n_0/n_3} \right\}^{-1}$$

mit einer Toleranz gleich oder kleiner 5%, vorzugsweise gleich oder kleiner 0,3% für $n_1$ und $k_1$, und mit einer Toleranz gleich oder kleiner 30% und vorzugsweise gleich oder kleiner 5% für $e_1$.

2. Kontrastverstärkungsträger nach Anspruch 1, wobei der Brechungsindex des Substrats $n_0$, der komplexe Brechungsindex der Schicht $N_1 = n_1 - jk_1$ und die Dicke $e_1$ der Schicht ferner mit den Toleranzen und für die Beleuchtungswellenlänge $\lambda$ die folgenden Bedingungen erfüllen:

a')

$$\nu_1^2 = 1 + \kappa_1^2 \ ;$$

b')

$$\delta_1 = \frac{n_0/n_3 - 1}{2\nu_1 \kappa_1};$$

und
c')

$$k_1 \geq 0,15_;$$

wobei:

$$\delta_1 = \frac{2\pi n_0}{\lambda} e_1;$$

$$v_1 = \frac{n_1}{\sqrt{n_0 n_3}} \; ;$$

-

$$\kappa_1 = \frac{k_1}{\sqrt{n_0 n_3}} \; ;$$

und

-

$$K = \left\{ [\pi / (n_0/n_3 - 1)] \sqrt{n_0/n_3} \right\}^{-1} .$$

3.  Kontrastverstärkungsträger nach einem der vorherigen Ansprüche, wobei der Träger aus Glas besteht.

4.  Kontrastverstärkungsträger nach Anspruch 3, wobei das Verhältnis $n_0/n_3$ zwischen 1,1 und 1,3 gewählt wird.

5.  Kontrastverstärkungsträger nach Anspruch 3, wobei das Verhältnis $n_0/n_3$ zwischen 1,45 und 1,7 gewählt wird.

6.  Kontrastverstärkungsträger nach einem der vorherigen Ansprüche, wobei die Schicht aus absorbierendem Material ausgewählt ist aus:

    - einer Schicht von in das Substrat implantierten Verunreinigungen;
    - einer metallischen Schicht;
    - einer Halbleiterschicht;
    - einer Metall/Halbleiter-Verbundlegierung;
    - einer Schicht aus magnetisch absorbierendem Material;
    - einer Schicht aus metallischen Nanopartikeln;
    - einer nicht-metallischen Leiterschicht;
    - einer diffundierenden Schicht;
    - einer Polymer- oder Photoresistschicht, die Pigmente oder Farbstoffe enthält;
    - einer dielektrischen Mineralschicht, die farbige Zentren enthält;
    - einer zusammengesetzten Hybridschicht, die eine kontinuierliche Phase umfasst, in der Nanopartikel dispergiert sind; und
    - einer Graphenschicht oder eine funktionalisierten Graphenschicht.

7.  Kontrastverstärkungsträger nach einem der vorherigen Ansprüche, bei dem die Schicht aus absorbierendem Material eine funktionalisierte Schicht umfasst, die mindestens eine chemische oder biologische Spezies fixieren kann.

8.  Verfahren zur Herstellung eines Kontrastverstärkungsträgers zum Beobachten einer auf den Träger abgesetzten Probe, mit einem transparenten Substrat (MI, S), das eine Schicht aus absorbierendem Material (CA) trägt, wobei das Verfahren eine Phase des Konzipierens des Trägers und eine Phase der Materialherstellung des so konzipierten Trägers beinhaltet; wobei der Konzeptionsschritt die folgenden Schritte beinhaltet:

    i) Wählen einer Beleuchtungswellenlänge $\lambda$;
    ii) Wählen eines Materials, das das Substrat bildet und bei der Beleuchtungswellenlänge $\lambda$ einen realen Brechungsindex $n_0$ aufweist;
    iii) Wählen eines Umgebungsmediums (ME) in Kontakt mit der Schicht auf der dem Substrat gegenüberliegenden Seite, das bei der Beleuchtungswellenlänge $\lambda$ einen realen Brechungsindex $n_3 < n_0$ aufweist;
    iv) Bestimmen eines nominalen komplexen Brechungsindex $N_1 = n_1 - jk_1$ und einer nominalen Dicke $e_1$ der Schicht, so dass sie sich als Antireflexionsschicht verhält, wenn sie bei normalem Einfall mit der Beleuchtungswellenlänge $\lambda$ durch das Substrat beleuchtet wird, wobei die dem Substrat gegenüberliegende Seite der Schicht in Kontakt mit dem Umgebungsmedium ist; und
    v) Wählen eines Materials, das die Schicht aus absorbierendem Material bildet und bei der Beleuchtungswel-

lenlänge $\lambda$ einen komplexen Brechungsindex aufweist, dessen Real- und Imaginärteil mit denen des nominalen komplexen Brechungsindexes mit einer Toleranz von gleich oder kleiner 5 % und vorzugsweise gleich oder kleiner 0,3 % übereinstimmen, wobei in Schritt iv) der nominale komplexe Brechungsindex und die nominale Dicke so gewählt werden, dass sie die folgenden Bedingungen erfüllen:

a)

$$v_1{}^2 = 1 + \kappa_1{}^2\ ;$$

b)

$$\delta_1 = \frac{(n_0/n_3 - 1)}{2v_1\kappa_1}\left[1 - e^{-\frac{\kappa_1}{K}}\right]$$

; und

c) $k_1 \geq 0{,}01$ und vorzugsweise $k_1 \geq 0{,}1$;

wobei

-

$$\delta_1 = \frac{2\pi n_0}{\lambda} e_1;$$

-

$$v_1 = \frac{n_1}{\sqrt{n_0 n_3}}\ ;$$

-

$$\kappa_1 = \frac{k_1}{\sqrt{n_0 n_3}}\ ;$$

und

-

$$K = \left\{[\pi/(n_0/n_3 - 1)]\sqrt{n_0/n_3}\right\}^{-1}.$$

**9.** Verfahren nach Anspruch 8, wobei der nominale komplexe Brechungsindex und die nominale Schichtdicke so bestimmt werden, dass für die Beleuchtungswellenlänge $\lambda$ die folgenden Bedingungen erfüllt sind:

a')

$$v_1{}^2 = 1 + \kappa_1{}^2\ ;$$

b')

$$\delta_1 = \frac{n_0/n_3 - 1}{2\nu_1\kappa_1};$$

und

c') $k_1 \geq 0{,}15$.

wobei:

-

$$\delta_1 = \frac{2\pi n_0}{\lambda} e_1;$$

-

$$\nu_1 = \frac{n_1}{\sqrt{n_0 n_3}};$$

-

$$\kappa_1 = \frac{k_1}{\sqrt{n_0 n_3}};$$

und

-

$$K = \left\{ [\pi/(n_0/n_3 - 1)]\sqrt{n_0/n_3} \right\}^{-1}.$$

**10.** Verfahren zum Beobachten einer Probe, das die folgenden Schritte beinhaltet:

A. Anordnen der Probe auf einer Schicht aus absorbierendem Material (CA) der Dicke $e_1$ mit einem komplexen Brechungsindex $N_1 = n_1 - jk_1$ zwischen einem ersten transparenten Einfallsmedium (MI, SS) mit einem realen Brechungsindex $n_0$ und einem zweiten transparenten Austrittsmedium (ME) mit einem realen Brechungsindex $n_3 < n_0$;

B. Beleuchten der Probe bei normalem Einfall mindestens mit der Beleuchtungswellenlänge $\lambda$ durch das Einfallsmedium;

C. Beobachten der so beleuchteten Probe, auch durch das Einfallsmedium; wobei die Schicht aus absorbierendem Material einen komplexen Brechungsindex und eine solche Dicke aufweist, dass:

a)

$$\nu_1{}^2 = 1 + \kappa_1{}^2;$$

b)

$$\delta_1 = \frac{(n_0/n_3 - 1)}{2\nu_1\kappa_1}\left[1 - e^{-\frac{\kappa_1}{K}}\right] \quad ;$$

und

c) $k_1 \geq 0{,}01$ und vorzugsweise $k_1 \geq 0{,}1$;

wobei

$$\delta_1 = \frac{2\pi n_0}{\lambda} e_1;$$

$$\nu_1 = \frac{n_1}{\sqrt{n_0 n_3}};$$

$$\kappa_1 = \frac{k_1}{\sqrt{n_0 n_3}};$$

und

$$K = \left\{ [\pi/(n_0/n_3 - 1)]\sqrt{n_0/n_3} \right\}^{-1};$$

mit einer Toleranz gleich oder kleiner 5%, vorzugsweise gleich oder kleiner 0,3% für $n_1$ und $k_1$, und mit einer Toleranz gleich oder kleiner 30%, vorzugsweise gleich oder kleiner 5% für $e_1$.

**11.** Verfahren nach Anspruch 10, wobei Schritt A mittels eines Kontrastverstärkungsträgers (SAC) nach einem der Ansprüche 1 bis 8 durchgeführt wird, dessen Substrat das Einfallsmedium darstellt.

**12.** Verfahren nach Anspruch 10 oder 11, wobei die Probe zumindest mit der Beleuchtungswellenlänge $\lambda$ durch einen parallelen oder fokussierten Lichtstrahl (FL) beleuchtet wird, so dass ein Beleuchtungskegel mit einem halben Öffnungswinkel gleich oder kleiner 20° gebildet wird.

**13.** Verfahren nach Anspruch 11, wobei die Probe zumindest mit der Beleuchtungswellenlänge $\lambda$ durch einen fokussierten Laserstrahl beleuchtet wird.

**14.** Verfahren zum Nachweisen oder Dosieren mindestens einer chemischen oder biologischen Spezies, das die folgenden Schritte beinhaltet:

    I. Bereitstellen eines Kontrastverstärkungsträgers (SAC) nach Anspruch 7, der eine funktionalisierte Schicht oder Oberfläche (CF) umfasst, die mindestens eine chemische oder biologische Spezies fixieren kann;
    II. Inkontaktbringen der funktionalisierten Schicht oder Oberfläche mit mindestens einer Lösung (S0, S0', S1, S1', S2), die eine chemische oder biologische Spezies enthält, die mit metallischen Nanopartikeln (NPM) oder einem absorbierenden oder diffundierenden Marker markiert ist, wobei sich die Spezies an die funktionalisierte Schicht oder Oberfläche binden kann, entweder direkt oder über eine oder mehrere andere chemische oder biologische Spezies, wodurch die Partikel eine kontinuierliche oder diskontinuierliche metallische, absorbierende oder diffundierende Schicht bilden;
    III. Beleuchten des Kontrastverstärkungsträgers bei normalem Einfall mindestens mit der Beleuchtungswellenlänge $\lambda$ durch das Substrat;
    IV. Beobachten des so beleuchteten Kontrastverstärkungsträgers, auch durch das Substrat.

**15.** Verfahren zum Nachweisen oder Dosieren mindestens einer chemischen oder biologischen Spezies, das die folgenden Schritte beinhaltet:

    I. Bereitstellen eines transparenten Substrats (SS), das eine funktionalisierte Schicht oder Oberfläche (CF) trägt, die mindestens eine chemische oder biologische Spezies fixieren kann;
    II. Inkontaktbringen der funktionalisierten Schicht oder Oberfläche mit mindestens einer Lösung (S0, S0', S1, S1', S2), die eine chemische oder biologische Spezies enthält, die mit metallischen Nanopartikeln (NPM) oder einem absorbierenden oder diffundierenden Marker markiert ist, wobei sich die Spezies an der funktionalisierten Schicht oder Oberfläche fixieren kann - entweder direkt, oder über eine oder mehrere andere chemische oder biologische Spezies - zur Bildung einer kontinuierlichen oder diskontinuierlichen metallischen, absorbierenden oder diffundierenden Schicht, wobei das transparente Substrat zusammen mit der funktionalisierten Schicht oder Oberfläche (CF) und der so gebildeten metallischen, absorbierenden oder diffundierenden Schicht einen Kontrastverstärkungsträger nach einem der Ansprüche 1 bis 5 bildet;

III. Beleuchten des Kontrastverstärkungsträgers bei normalem Einfall mindestens mit der Beleuchtungswellenlänge λ durch das Substrat;

IV. Beobachten des so beleuchteten Kontrastverstärkungsträgers, auch durch das Substrat.

**16.** Verfahren zum Nachweisen oder Dosieren mindestens einer chemischen oder biologischen Spezies nach Anspruch 15, wobei Schritt II die folgenden Unterschritte beinhaltet:

II-1) Inkontaktbringen der funktionalisierten Schicht oder Oberfläche mit einer ersten Lösung (S1), die die nachzuweisende oder zu dosierende chemische oder biologische Spezies (ECD) enthält, um so eine Zwischenschicht (CI) zu bilden; und

II-2) Inkontaktbringen der Zwischenschicht mit einer zweiten Lösung (S2), die eine chemische oder biologische Hilfsspezies (ECA) enthält, die durch metallische Nanopartikel oder einen absorbierenden oder diffundierenden Marker gekennzeichnet ist und sich an der Zwischenschicht fixieren kann, um die kontinuierliche oder diskontinuierliche metallische, absorbierende oder diffundierende Schicht zu bilden.

**17.** Verfahren zum Nachweisen oder Dosieren mindestens einer chemischen oder biologischen Spezies nach Anspruch 15, wobei Schritt II die folgenden Unterschritte beinhaltet:

II-1') Inkontaktbringen der funktionalisierten Schicht oder Oberfläche mit einer ersten Lösung (S1'), die eine chemische oder biologische Zwischenspezies (ECI) enthält, die durch metallische Nanopartikel oder einen absorbierenden oder diffundierenden Marker markiert ist und sich an die Funktionalisierungsschicht fixieren kann, um die kontinuierliche oder diskontinuierliche metallische, absorbierende oder diffundierende Schicht zu bilden; und

II-2') Inkontaktbringen der funktionalisierten Schicht oder Oberfläche und der metallischen, absorbierenden oder diffundierenden Schicht mit einer zweiten Lösung (S2), die die nachzuweisende oder zu dosierende chemische oder biologische Spezies enthält, die eine Affinität zu der funktionalisierten Schicht oder Oberfläche hat, die größer ist als die der Zwischenspezies, wodurch die Zwischenspezies verdrängt und die metallische, absorbierende oder diffundierende Schicht zumindest teilweise entfernt wird.

**18.** Verfahren zum Dosieren mindestens einer chemischen oder biologischen Spezies nach Anspruch 15, wobei in Schritt II die funktionalisierte Schicht oder Oberfläche mit einer Lösung (S0') in Kontakt gebracht wird, die die zu dosierende chemische oder biologische Spezies (ECD) sowie die konkurrierende chemische oder biologische Spezies (ECC) enthält, wobei eine der beiden Spezies mit metallischen Nanopartikeln oder einem absorbierenden oder diffundierenden Marker markiert wird, wodurch eine kontinuierliche oder diskontinuierliche metallische, absorbierende oder diffundierende Schicht erhalten wird, deren effektive Dicke vom Verhältnis zwischen der Konzentration der konkurrierenden chemischen oder biologischen Spezies und der der zu dosierenden chemischen oder biologischen Spezies abhängt.

## Claims

**1.** A contrast-amplifying support (SAC) for the observation of a sample, deposited on said support, comprising a transparent substrate (MI, SS) carrying an absorbing layer (CA) whose complex refractive index $N_1 = n_1 - jk_1$ and thickness are chosen in such a way that said layer behaves in the guise of antireflection layer when it is illuminated under normal incidence at an illumination wavelength λ through said substrate, the face of said layer opposite to said substrate being in contact with a transparent so-called ambient medium (ME) whose refractive index $n_3$ is less than that of the refractive index $n_0$ of said substrate and in which, at said illumination wavelength λ, the refractive index $n_0$ of the substrate, the real and imaginary parts of the complex refractive index of the layer $N_1 = n_1 - jk_1$ and the thickness $e_1$ of the layer satisfy the following conditions:

a)

$$\nu_1{}^2 = 1 + \kappa_1{}^2 \,;$$

b)

$$\delta_1 = \frac{(n_0/n_3 - 1)}{2\nu_1\kappa_1}\left[1 - e^{-\frac{\kappa_1}{K}}\right] ;$$

and
c) $\kappa_1 \geq 0.01$ and preferably $\kappa_1 \geq 0.1$,
where:

-

$$\delta_1 = \frac{2\pi n_0}{\lambda}e_1;$$

-

$$\nu_1 = \frac{n_1}{\sqrt{n_0 n_3}} ;$$

-

$$\kappa_1 = \frac{k_1}{\sqrt{n_0 n_3}} ;$$

and
-

$$K = \left\{[\pi/(n_0/n_3 - 1)]\sqrt{n_0/n_3}\right\}^{-1}$$

with a tolerance of less than or equal to 5%, and preferably less than or equal to 0.3%, for $n_1$ and $k_1$, and with a tolerance of less than or equal to 30% and preferably less than or equal to 5% for $e_1$.

2. The contrast-amplifying support according to claim 1, in which the refractive index of the substrate $n_0$, the complex refractive index of the layer $N_1 = n_1 - jk_1$ and the thickness $e_1$ of the layer satisfy, furthermore, with said tolerances and for said illumination wavelength $\lambda$, the following conditions:

a')

$$\nu_1{}^2 = 1 + \kappa_1{}^2 ;$$

b')

$$\delta_1 = \frac{n_0/n_3 - 1}{2\nu_1\kappa_1};$$

and
c') $\kappa_1 \geq 0.15$
where:

-

$$\delta_1 = \frac{2\pi n_0}{\lambda} e_1;$$

-

$$\nu_1 = \frac{n_1}{\sqrt{n_0 n_3}};$$

-

$$\kappa_1 = \frac{k_1}{\sqrt{n_0 n_3}};$$

and

-

$$K = \left\{ [\pi/(n_0/n_3 - 1)] \sqrt{n_0/n_3} \right\}^{-1}.$$

3. The contrast-amplifying support according to one of the preceding claims, in which said substrate is made of glass.

4. The contrast-amplifying support according to claim 3, in which the ratio $n_0/n_3$ is chosen to lie between 1.1 and 1.3.

5. The contrast-amplifying support according to claim 3, in which the ratio $n_0/n_3$ is chosen to lie between 1.45 and 1.7.

6. The contrast-amplifying support according to one of the preceding claims, in which said absorbing layer is chosen from among:

   - a layer of impurities implanted in said substrate;
   - a metallic layer;
   - a semi-conductor layer;
   - a metal/semi-conductor composite alloy;
   - a magnetic absorbing layer;
   - a layer of metallic nanoparticles;
   - a non-metallic conducting layer;
   - a diffusing layer;
   - a polymer or photoresist layer containing pigments or dyes;
   - a mineral dielectric layer containing color centers;
   - a composite hybrid layer comprising a continuous phase in which nanoparticles are dispersed; and
   - a graphene layer or a functionalized graphene layer.

7. The contrast-amplifying support according to one of the preceding claims, in which said absorbing layer comprises at least one functionalized layer, able to fix at least one chemical or biological species.

8. A method for producing a contrast-amplifying support for the observation of a sample, deposited on said support comprising a transparent substrate (MI, S) carrying an absorbing layer (CA), said method comprising a design phase for said support and a phase of hardware production of the support thus designed, in which said design step comprises the following steps:

   i) choosing an illumination wavelength $\lambda$;
   ii) choosing a material constituting said substrate and exhibiting, at said illumination wavelength $\lambda$, a real refractive index $n_0$;
   iii) choosing an ambient medium (ME) in contact with said layer on the side opposite to said substrate and exhibiting, at said illumination wavelength $\lambda$, a real refractive index $n_3 < n_0$;

iv) determining a nominal complex refractive index $N_1 = n_1 - jk_1$ and a nominal thickness $e_1$ of said layer which are such that it behaves in the guise of antireflection layer when it is illuminated under normal incidence at said illumination wavelength $\lambda$ through said substrate, the face of said layer opposite to said substrate being in contact with said ambient medium; and

v) choosing a material constituting said absorbing layer and exhibiting, at said illumination wavelength $\lambda$, a complex refractive index whose real and imaginary parts coincide with those of said nominal complex refractive index at least by a tolerance of less than or equal to 5%, and preferably less than or equal to 0.3%, in which, during said step iv), said nominal complex refractive index and said nominal thickness are chosen satisfying the following conditions:

a)

$$v_1{}^2 = 1 + \kappa_1{}^2 \, ;$$

b)

$$\delta_1 = \frac{(n_0/n_3 - 1)}{2 v_1 \kappa_1} \left[ 1 - e^{-\frac{\kappa_1}{K}} \right]$$

; and

c) $\kappa_1 \geq 0.01$ and preferably $\kappa_1 \geq 0.1$,
where:

-

$$\delta_1 = \frac{2\pi n_0}{\lambda} e_1 ;$$

-

$$v_1 = \frac{n_1}{\sqrt{n_0 n_3}} \ ;$$

-

$$\kappa_1 = \frac{k_1}{\sqrt{n_0 n_3}} \ ;$$

and

-

$$K = \left\{ [\pi/(n_0/n_3 - 1)] \sqrt{n_0/n_3} \right\}^{-1}$$

.

9. The method according to claim 8, in which the nominal complex refractive index and the nominal thickness of the layer are determined so as to satisfy, for said illumination wavelength $\lambda$, the following conditions:

a')

$$v_1{}^2 = 1 + \kappa_1{}^2 \ ;$$

b')

$$\delta_1 = \frac{n_0/n_3 - 1}{2v_1\kappa_1};$$

and

c') $\kappa_1 \geq 0.15$

where:

-

$$\delta_1 = \frac{2\pi n_0}{\lambda} e_1;$$

-

$$v_1 = \frac{n_1}{\sqrt{n_0 n_3}};$$

-

$$\kappa_1 = \frac{k_1}{\sqrt{n_0 n_3}};$$

and

-

$$K = \left\{ [\pi/(n_0/n_3 - 1)]\sqrt{n_0/n_3} \right\}^{-1}.$$

**10.** A method for observing a sample comprising the following steps:

A. disposing said sample on an absorbing layer (CA) of thickness $e_1$ exhibiting a complex refractive index $N_1 = n_1 - jk_1$, lying between a first, so-called incident, transparent medium (MI, SS), having a real refractive index $n_0$ and a second, so-called emergent, transparent medium (ME), having a real refractive index $n_3 < n_0$;

B. illuminating said sample under normal incidence at least at said illumination wavelength $\lambda$ through said incident medium;

C. observing the sample thus illuminated, also through said incident medium; in which said absorbing layer exhibits a complex refractive index and a thickness which are such that:

a)

$$v_1^2 = 1 + \kappa_1^2;$$

b)

$$\delta_1 = \frac{(n_0/n_3 - 1)}{2v_1\kappa_1}\left[1 - e^{-\frac{\kappa_1}{K}}\right];$$

and

c) $\kappa_1 \geq 0.01$ and preferably $\kappa_1 \geq 0.1$,
where:

$$\delta_1 = \frac{2\pi n_0}{\lambda} e_1;$$

$$\nu_1 = \frac{n_1}{\sqrt{n_0 n_3}};$$

$$\kappa_1 = \frac{k_1}{\sqrt{n_0 n_3}};$$

and

$$K = \left\{ [\pi/(n_0/n_3 - 1)]\sqrt{n_0/n_3} \right\}^{-1}$$

with a tolerance of less than or equal to 5%, and preferably less than or equal to 0.3%, for $n_0$, $n_1$ and $k_1$ and with a tolerance of less than or equal to 30% and preferably less than or equal to 5% for $e_1$.

**11.** The method according to claim 10, in which said step A is implemented by means of a contrast-amplifying support (SAC) according to one of claims 1 to 8, whose substrate constitutes said incident medium.

**12.** The method according either of claims 10 or 11, in which said sample is illuminated at least at said illumination wavelength $\lambda$ by a parallel or focused light beam (FL) so as to form an illumination cone exhibiting a semi-aperture angle of less than or equal to 20°.

**13.** The method according to claim 11, in which said sample is illuminated at least at said illumination wavelength $\lambda$ by a focused laser beam.

**14.** A method for detecting or assaying at least one chemical or biological species comprising the following steps:

I. procuring a contrast-amplifying support (SAC) according to claim 7, comprising a functionalized layer or surface (CF), able to fix at least one chemical or biological species;
II. placing said functionalized layer or surface in contact with at least one solution (S0, S0', S1, S1', S2) containing a chemical or biological species marked by metallic nanoparticles (NPM) or an absorbing or diffusing marker, said species being able to be fixed on said functionalized layer or surface, either directly, or by way of one or more other chemical or biological species, whereby said particles form a continuous or discontinuous, absorbing or diffusing metallic layer;
III. illuminating said contrast-amplifying support under normal incidence at least at said illumination wavelength $\lambda$ through said substrate;
IV. observing said contrast-amplifying support thus illuminated, also through said substrate.

**15.** A method for detecting or assaying at least one chemical or biological species comprising the following steps:

I. procuring a transparent substrate (SS) carrying a functionalized layer or surface (CF), able to fix at least one chemical or biological species;

II. placing said functionalized layer or surface in contact with at least one solution (S0, S0', S1, S1', S2) containing a chemical or biological species marked by metallic nanoparticles (NPM) or an absorbing or diffusing marker, said species being able to be fixed on said functionalized layer or surface - either directly, or by way of one or more other chemical or biological species - so as to form a continuous or discontinuous, absorbing or diffusing metallic layer, said transparent substrate forming, with said functionalized layer or surface (CF) and the absorbing or diffusing metallic layer thus formed, a contrast-amplifying support according to one of claims 1 to 5;

III. illuminating said contrast-amplifying support under normal incidence at least at said illumination wavelength λ through said substrate;

IV. observing said contrast-amplifying support thus illuminated, also through said substrate.

16. The method for detecting or assaying at least one chemical or biological species according to claim 15, in which said step II comprises the sub-steps consisting in:

II-1) Placing said functionalized layer or surface in contact with a first solution (S1) containing the chemical or biological species to be detected or assayed (ECD), so as to form a so-called intermediate layer (CI); and

II-2) Placing said intermediate layer in contact with a second solution (S2), containing a so-called auxiliary chemical or biological species (ECA), marked by metallic nanoparticles or an absorbing or diffusing marker and able to be fixed on said intermediate layer so as to form said continuous or discontinuous, absorbing or diffusing metallic layer.

17. The method for detecting or assaying at least one chemical or biological species according to claim 15, in which said step II comprises the sub-steps consisting in:

II-1') Placing said functionalized layer or surface in contact with a first solution (S1') containing a chemical or biological species, the so-called intermediate species (ECI), marked by metallic nanoparticles or an absorbing or diffusing marker and able to be fixed on said functionalization layer so as to form said continuous or discontinuous, absorbing or diffusing metallic layer; and

II-2') Placing said functionalized layer or surface and said absorbing or diffusing metallic layer in contact with a second solution (S2) containing said chemical or biological species to be detected or assayed, which exhibits an affinity with said functionalized layer or surface which is greater than that of said intermediate species, whereby said intermediate species is displaced and said absorbing or diffusing metallic layer is removed at least in part.

18. The method for assaying at least one chemical or biological species according to claim 15, in which, during said step II, said functionalized layer or surface is placed in contact with a solution (S0') containing the chemical or biological species to be assayed (ECD), as well as said competing chemical or biological species (ECC), one of the two species being marked by metallic nanoparticles or an absorbing or diffusing marker, whereby a continuous or discontinuous, absorbing or diffusing metallic layer is obtained whose effective thickness depends on the ratio between the concentration of said competing chemical or biological species and that of said chemical or biological species to be assayed.

ME    $n_3$

CA    $N_1 = n_1 - jk_1$    $e_1$

MI    $n_0$

$\lambda$

FL

## FIG.1

## FIG.2A

## FIG.2B

FIG.2C

FIG.2D

FIG.3A

FIG.3B

FIG.3C

FIG.4A

FIG.4B

FIG.4C

FIG.4D

FIG.4E

FIG.4F

FIG.5A

FIG.5B

FIG.5C

FIG.5D

FIG.6

CE ECD

S CF

CA

ST

FIG.7A

CA' FL

SAC'

SA

FIG.8

FIG.7B

FIG.7C

FIG.7D

FIG.7E

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5216542 A

**Littérature non-brevet citée dans la description**

- **S. G. MOISEEV ; S. V. VINOGRADOV.** Design of Antireflection Composite Coating Based on Metal Nanoparticle. *Physics of Wave Phenomena,* 2011, vol. 10 (1), 47-51
- **M. A. KATS et al.** Nanometre optical coatings based on strong interference effects in higly absorbing média. *Nature Materials,* Janvier 2013, vol. 12, 20-24
- **R. M. A. AZZAM et al.** Antieflection of an absorbing substrate by an absorbing thin film at normal incidence. *Applied Optics,* 1987, vol. 26 (4), 719-722